(19) Europäisches Patentamt
European Patent Office
Office européen des brevets

(11) EP 4 763 206 A1

(12) EUROPEAN PATENT APPLICATION
published in accordance with Art. 153(4) EPC

(43) Date of publication:
**24.06.2026 Bulletin 2026/26**

(21) Application number: **24856264.7**

(22) Date of filing: **05.08.2024**

(51) International Patent Classification (IPC):
***A61K 31/42*** (2006.01) ***A61K 31/422*** (2006.01)
***A61K 31/4152*** (2006.01) ***A61P 21/00*** (2006.01)
***A61P 25/00*** (2006.01) ***A61P 43/00*** (2006.01)

(52) Cooperative Patent Classification (CPC):
**A61K 31/4152; A61K 31/42; A61K 31/422; A61P 21/00; A61P 25/00; A61P 43/00**

(86) International application number:
**PCT/JP2024/027845**

(87) International publication number:
**WO 2025/041572 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.08.2023 JP 2023133190**

(71) Applicant: **Socium Inc.**
**Tokyo, 103-0026 (JP)**

(72) Inventor: **HORIMOTO, Katsuhisa**
**Tokyo 103-0026 (JP)**

(74) Representative: **De Clercq & Partners**
**Edgard Gevaertdreef 10a**
**9830 Sint-Martens-Latem (BE)**

# (54) AGENT FOR SUPPRESSING AGGREGATION OF TDP-43, AGENT FOR SUPPRESSING CELL DEATH OF CELLS IN WHICH TDP-43 IS OVEREXPRESSED, AND AGENT FOR PREVENTING OR TREATING DISEASES ASSOCIATED WITH AGGREGATION OF TDP-43

(57) A suppressor of TDP-43 aggregation includes a combination of at least one selected from the group consisting of cycloserine, terizidone, and salts thereof, and edaravone. A suppressor of TDP-43 aggregation includes at least one selected from the group consisting of cycloserine, terizidone, and salts thereof, used in combination with edaravone. A suppressor of TDP-43 aggregation includes edaravone used in combination with at least one selected from the group consisting of cycloserine, terizidone, and salts thereof.

**Fig. 11**

| Edaravone (nmol/L) | 0 | 10 | 30 | 100 | 300 | 1000 |
|---|---|---|---|---|---|---|
| D-cycloserine EC50 (nmol/L) | 151.36 | 149.44 | 135.18 | 118.85 | 106.76 | 87.62 |
| Combination Index | | 0.99 | 0.91 | 0.83 | 0.83 | 1.01 |



Processed by Luminess, 75001 PARIS (FR)

## Description

### Technical Field

The present invention relates to a suppressor of TDP-43 aggregation, a suppressor of cell death in TDP-43-overexpressing cells, and an agent for treating or preventing diseases associated with TDP-43 aggregation.

### Background Art

TDP-43 (TAR DNA-binding protein of 43kDa) is a type of heterogeneous nuclear ribonucleoprotein. TDP-43 has been identified as a major structural protein of ubiquitin-positive inclusions that appear in degenerated nerve cells and glial cells in amyotrophic lateral sclerosis (ALS) and frontotemporal lobar degeneration (FTLD). TDP-43 is a nuclear-localized protein, but in cells in which ubiquitin-positive inclusions are formed, TDP-43 translocates from the nucleus to the cytoplasm, aggregates, becomes insoluble, and accumulates in the cytoplasm.

TDP-43, which accumulates in the brains and spinal cords of ALS patients and FTLD patients, is abnormally phosphorylated at a plurality of serine residues at the C-terminal. In ALS patients and FTLD patients, it is not necessary for the full length of TDP-43 molecules to aggregate, rather, C-terminal fragments of TDP-43 aggregate. Previous studies have strongly suggested that TDP-43 aggregation causes abnormalities in RNA metabolism and induces cytotoxicity, leading to the onset and progression of various diseases.

As an example of diseases associated with TDP-43 aggregation, TDP-43 proteinopathy is known. Patent Literature 1 proposes the use of N,N,N',N'-tetramethyl-10H-phenothiazine-3,7-diaminium bis(methanesulfonate) as an agent for treating or preventing TDP-43 proteinopathy.

### Citation List

### Patent Literature

Patent Literature 1: Japanese Patent No. 5898701

### Summary of Invention

### Technical Problem

One object of the present invention is to provide a suppressor of TDP-43 aggregation, a suppressor of cell death in TDP-43-overexpressing cells, and an agent for treating or preventing diseases associated with TDP-43 aggregation.

### Solution to Problem

An aspect of the present invention provides a suppressor of TDP-43 aggregation or suppressor of cell death in TDP-43-overexpressing cells, the suppressor including a combination of at least one selected from the group consisting of cycloserine, terizidone, and salts thereof, and edaravone.

In the suppressor of TDP-43 aggregation or suppressor of cell death in TDP-43-overexpressing cells, the active component may essentially consist of a combination of at least one selected from the group consisting of cycloserine, terizidone, and salts thereof, and edaravone.

In the present disclosure, "essentially consists of" means that the active component does not include other components, but the presence of components other than the active component, such as excipients and/or lubricants, is not excluded.

In the suppressor of TDP-43 aggregation or suppressor of cell death in TDP-43-overexpressing cells, the active component may be a combination of at least one selected from the group consisting of cycloserine, terizidone, and salts thereof, and edaravone.

In the suppressor of TDP-43 aggregation or suppressor of cell death in TDP-43-overexpressing cells, at least one selected from the group consisting of cycloserine, terizidone, and salts thereof may be cycloserine or salt thereof.

In the suppressor of TDP-43 aggregation or suppressor of cell death in TDP-43-overexpressing cells, the cycloserine may be D-cycloserine. In the suppressor of TDP-43 aggregation or suppressor of cell death in TDP-43-overexpressing cells, the cycloserine may be L-cycloserine.

The suppressor of TDP-43 aggregation or suppressor of cell death in TDP-43-overexpressing cells may include 15 mg or more and less than 250 mg of at least one selected from the group consisting of cycloserine, terizidone, and salts thereof.

The suppressor of TDP-43 aggregation or suppressor of cell death in TDP-43-overexpressing cells may include 30 mg or more and 60 mg or less of edaravone.

The suppressor of TDP-43 aggregation or suppressor of cell death in TDP-43-overexpressing cells may not include acamprosate.

An aspect of the present invention provides a combination of at least one selected from the group consisting of cycloserine, terizidone, and salts thereof, and edaravone, which is used to inhibit TDP-43 aggregation or inhibit cell death in TDP-43-overexpressing cells.

In the combination, the active component may essentially consist of a combination of at least one selected from the group consisting of cycloserine, terizidone, and salts thereof, and edaravone.

In the combination, the active component may be a combination of at least one selected from the group consisting of cycloserine, terizidone, and salts thereof,

and edaravone.

In the combination, at least one selected from the group consisting of cycloserine, terizidone, and salts thereof may be cycloserine or salt thereof.

In the combination, the cycloserine may be D-cycloserine. In the combination, the cycloserine may be L-cycloserine.

The combination may include 15 mg or more and less than 250 mg of at least one selected from the group consisting of cycloserine, terizidone, and salts thereof.

The combination may include 30 mg or more and 60 mg or less of edaravone.

The combination may not include acamprosate.

An aspect of the present invention provides a use of a combination of at least one selected from the group consisting of cycloserine, terizidone, and salts thereof, and edaravone, which is used to produce a suppressor of TDP-43 aggregation or a suppressor of cell death in TDP-43-overexpressing cells.

In the use, the active component of the suppressor of TDP-43 aggregation or suppressor of cell death in TDP-43-overexpressing cells may essentially consist of a combination of at least one selected from the group consisting of cycloserine, terizidone, and salts thereof, and edaravone.

In the use, the active component of the suppressor of TDP-43 aggregation or suppressor of cell death in TDP-43-overexpressing cells may be a combination of at least one selected from the group consisting of cycloserine, terizidone, and salts thereof, and edaravone.

In the use, at least one selected from the group consisting of cycloserine, terizidone, and salts thereof may be cycloserine or salt thereof.

In the use, the cycloserine may be D-cycloserine. In the use, the cycloserine may be L-cycloserine.

In the use, the combination may include 15 mg or more and less than 250 mg of at least one selected from the group consisting of cycloserine, terizidone, and salts thereof.

In the use, the combination may include 30 mg or more and 60 mg or less of edaravone.

In the use, the suppressor of TDP-43 aggregation or suppressor of cell death in TDP-43-overexpressing cells may not include acamprosate.

An aspect of the present invention provides a method of inhibiting TDP-43 aggregation in a subject or method of inhibiting cell death in TDP-43-overexpressing cells in a subject, the method including administering a combination of at least one selected from the group consisting of cycloserine, terizidone, and salts thereof, and edaravone to a subject.

In the method of inhibiting TDP-43 aggregation or method of inhibiting cell death in TDP-43-overexpressing cells, the active component that inhibits TDP-43 aggregation may essentially consist of a combination of at least one selected from the group consisting of cycloserine, terizidone, and salts thereof, and edaravone.

In the method of inhibiting TDP-43 aggregation or method of inhibiting cell death in TDP-43-overexpressing cells, the active component that inhibits TDP-43 aggregation may be a combination of at least one selected from the group consisting of cycloserine, terizidone, and salts thereof, and edaravone.

In the method of inhibiting TDP-43 aggregation or method of inhibiting cell death in TDP-43-overexpressing cells, at least one selected from the group consisting of cycloserine, terizidone, and salts thereof may be cycloserine or salt thereof.

In the method of inhibiting TDP-43 aggregation or method of inhibiting cell death in TDP-43-overexpressing cells, the cycloserine may be D-cycloserine. In the method of inhibiting TDP-43 aggregation or method of inhibiting cell death in TDP-43-overexpressing cells, the cycloserine may be L-cycloserine.

In the method of inhibiting TDP-43 aggregation or method of inhibiting cell death in TDP-43-overexpressing cells, a single dose of at least one selected from the group consisting of cycloserine, terizidone, and salts thereof may be 15 mg or more and less than 250 mg.

In the method of inhibiting TDP-43 aggregation or method of inhibiting cell death in TDP-43-overexpressing cells, a single dose of edaravone may be 30 mg or more and 60 mg or less.

In the method of inhibiting TDP-43 aggregation or method of inhibiting cell death in TDP-43-overexpressing cells, acamprosate may not be administered to a subject.

An aspect of the present invention provides a suppressor of TDP-43 aggregation or suppressor of cell death in TDP-43-overexpressing cells, the suppressor including at least one selected from the group consisting of cycloserine, terizidone, and salts thereof, used in combination with edaravone.

In the suppressor of TDP-43 aggregation or suppressor of cell death in TDP-43-overexpressing cells, at least one selected from the group consisting of cycloserine, terizidone, and salts thereof may not be used in combination with an active component other than edaravone.

In the suppressor of TDP-43 aggregation or suppressor of cell death in TDP-43-overexpressing cells, at least one selected from the group consisting of cycloserine, terizidone, and salts thereof may be cycloserine or salt thereof.

In the suppressor of TDP-43 aggregation or suppressor of cell death in TDP-43-overexpressing cells, the cycloserine may be D-cycloserine. In the suppressor of TDP-43 aggregation or suppressor of cell death in TDP-43-overexpressing cells, the cycloserine may be L-cycloserine.

The suppressor of TDP-43 aggregation or suppressor of cell death in TDP-43-overexpressing cells may include 15 mg or more and less than 250 mg of at least

one selected from the group consisting of cycloserine, terizidone, and salts thereof.

In the suppressor of TDP-43 aggregation or suppressor of cell death in TDP-43-overexpressing cells, at least one selected from the group consisting of cycloserine, terizidone, and salts thereof may be used in combination with 30 mg or more and 60 mg or less of edaravone.

The suppressor of TDP-43 aggregation or suppressor of cell death in TDP-43-overexpressing cells may not include acamprosate.

An aspect of the present invention provides at least one selected from the group consisting of cycloserine, terizidone, and salts thereof, used in combination with edaravone, which is used to inhibit TDP-43 aggregation or inhibit cell death in TDP-43-overexpressing cells.

The at least one selected from the group consisting of cycloserine, terizidone, and salts thereof may not be used in combination with an active component other than edaravone.

The at least one selected from the group consisting of cycloserine, terizidone, and salts thereof may be cycloserine or salt thereof.

In the at least one selected from the group consisting of cycloserine, terizidone, and salts thereof, the cycloserine may be D-cycloserine. In the at least one selected from the group consisting of cycloserine, terizidone, and salts thereof, the cycloserine may be L-cycloserine.

The mass of the at least one selected from the group consisting of cycloserine, terizidone, and salts thereof may be 15 mg or more and less than 250 mg.

The mass of edaravone used in combination with the at least one selected from the group consisting of cycloserine, terizidone, and salts thereof may be 30 mg or more and less than 60 mg.

The edaravone may not be used in combination with acamprosate.

An aspect of the present invention provides a use of at least one selected from the group consisting of cycloserine, terizidone, and salts thereof, used in combination with edaravone, which is used to produce a suppressor of TDP-43 aggregation or a suppressor of cell death in TDP-43-overexpressing cells.

In the use, the at least one selected from the group consisting of cycloserine, terizidone, and salts thereof may not be used in combination with an active component other than edaravone.

In the use, the at least one selected from the group consisting of cycloserine, terizidone, and salts thereof may be cycloserine or salt thereof.

In the use, the cycloserine may be D-cycloserine. In the use, the cycloserine may be L-cycloserine.

In the use, the mass of the at least one selected from the group consisting of cycloserine, terizidone, and salts thereof may be 15 mg or more and less than 250 mg.

In the use, the mass of edaravone used in combination with at least one selected from the group consisting of cycloserine, terizidone, and salts thereof may be 30 mg or more and less than 60 mg.

In the use, the at least one selected from the group consisting of cycloserine, terizidone, and salts thereof may not be used in combination with acamprosate.

An aspect of the present invention provides a method of inhibiting TDP-43 aggregation in a subject or method of inhibiting cell death in TDP-43-overexpressing cells in a subject, the method including administering at least one selected from the group consisting of cycloserine, terizidone, and salts thereof, used in combination with edaravone to a subject.

In the method of inhibiting TDP-43 aggregation or method of inhibiting cell death in TDP-43-overexpressing cells, the at least one selected from the group consisting of cycloserine, terizidone, and salts thereof may not be used in combination with an active component other than edaravone.

In the method of inhibiting TDP-43 aggregation or method of inhibiting cell death in TDP-43-overexpressing cells, the at least one selected from the group consisting of cycloserine, terizidone, and salts thereof may be cycloserine or salt thereof.

In the method of inhibiting TDP-43 aggregation or method of inhibiting cell death in TDP-43-overexpressing cells, the cycloserine may be D-cycloserine. In the method of inhibiting TDP-43 aggregation or method of inhibiting cell death in TDP-43-overexpressing cells, the cycloserine may be L-cycloserine.

In the method of inhibiting TDP-43 aggregation or method of inhibiting cell death in TDP-43-overexpressing cells, a single dose of at least one selected from the group consisting of cycloserine, terizidone, and salts thereof may be 15 mg or more and less than 250 mg.

In the method of inhibiting TDP-43 aggregation or method of inhibiting cell death in TDP-43-overexpressing cells, the at least one selected from the group consisting of cycloserine, terizidone, and salts thereof may be used in combination with 30 mg or more and 60 mg or less of edaravone.

In the method of inhibiting TDP-43 aggregation or method of inhibiting cell death in TDP-43-overexpressing cells, acamprosate may not be administered to a subject.

An aspect of the present invention provides a suppressor of TDP-43 aggregation or suppressor of cell death in TDP-43-overexpressing cells, the method including edaravone used in combination with at least one selected from the group consisting of cycloserine, terizidone, and salts thereof.

In the suppressor of TDP-43 aggregation or suppressor of cell death in TDP-43-overexpressing cells, edaravone may not be used in combination with an active component other than at least one selected from the group consisting of cycloserine, terizidone, and salts thereof.

In the suppressor of TDP-43 aggregation or suppressor of cell death in TDP-43-overexpressing cells, the at least one selected from the group consisting of cycloserine, terizidone, and salts thereof may be cycloserine or salt thereof.

In the suppressor of TDP-43 aggregation or suppressor of cell death in TDP-43-overexpressing cells, the cycloserine may be D-cycloserine. In the suppressor of TDP-43 aggregation or suppressor of cell death in TDP-43-overexpressing cells, the cycloserine may be L-cycloserine.

In the suppressor of TDP-43 aggregation or suppressor of cell death in TDP-43-overexpressing cells, edaravone may be used in combination with 15 mg or more and less than 250 mg of at least one selected from the group consisting of cycloserine, terizidone, and salts thereof.

The suppressor of TDP-43 aggregation or suppressor of cell death in TDP-43-overexpressing cells may include 30 mg or more and 60 mg or less of edaravone.

The suppressor of TDP-43 aggregation or suppressor of cell death in TDP-43-overexpressing cells may not include acamprosate.

An aspect of the present invention provides edaravone used in combination with at least one selected from the group consisting of cycloserine, terizidone, and salts thereof, which is used to inhibit TDP-43 aggregation or inhibit cell death in TDP-43-overexpressing cells.

The edaravone may not be used in combination with an active component other than at least one selected from the group consisting of cycloserine, terizidone, and salts thereof.

The at least one selected from the group consisting of cycloserine, terizidone, and salts thereof used in combination with edaravone may be cycloserine or salt thereof.

The cycloserine used in combination with edaravone may be D-cycloserine. The cycloserine used in combination with edaravone may be L-cycloserine.

The edaravone may be used in combination with 15 mg or more and less than 250 mg of at least one selected from the group consisting of cycloserine, terizidone, and salts thereof.

The mass of edaravone may be 30 mg or more and 60 mg or less.

The at least one selected from the group consisting of cycloserine, terizidone, and salts thereof used in combination with edaravone may not be used in combination with acamprosate.

An aspect of the present invention provides a use of edaravone used in combination with at least one selected from the group consisting of cycloserine, terizidone, and salts thereof, which is used to produce a suppressor of TDP-43 aggregation or a suppressor of cell death in TDP-43-overexpressing cells.

In the use, edaravone may not be used in combination with an active component other than at least one selected from the group consisting of cycloserine, terizidone, and salts thereof.

In the use, the at least one selected from the group consisting of cycloserine, terizidone, and salts thereof may be cycloserine or salt thereof.

In the use, the cycloserine may be D-cycloserine. In the use, the cycloserine may be L-cycloserine.

In the use, the mass of the at least one selected from the group consisting of cycloserine, terizidone, and salts thereof used in combination with edaravone may be 15 mg or more and less than 250 mg.

In the use, the mass of edaravone may be 30 mg or more and less than 60 mg.

In the use, edaravone may not be used in combination with acamprosate.

An aspect of the present invention provides a method of inhibiting TDP-43 aggregation in a subject or method of inhibiting cell death in TDP-43-overexpressing cells in a subject, the method including administering edaravone to a subject used in combination with at least one selected from the group consisting of cycloserine, terizidone, and salts thereof.

In the method of inhibiting TDP-43 aggregation or method of inhibiting cell death in TDP-43-overexpressing cells, edaravone may not be used in combination with an active component other than at least one selected from the group consisting of cycloserine, terizidone, and salts thereof.

In the method of inhibiting TDP-43 aggregation or method of inhibiting cell death in TDP-43-overexpressing cells, the at least one selected from the group consisting of cycloserine, terizidone, and salts thereof may be cycloserine or salt thereof.

In the method of inhibiting TDP-43 aggregation or method of inhibiting cell death in TDP-43-overexpressing cells, the cycloserine may be D-cycloserine. In the method of inhibiting TDP-43 aggregation or method of inhibiting cell death in TDP-43-overexpressing cells, the cycloserine may be L-cycloserine.

In the method of inhibiting TDP-43 aggregation or method of inhibiting cell death in TDP-43-overexpressing cells, a single dose of at least one selected from the group consisting of cycloserine, terizidone, and salts thereof used in combination with edaravone may be 15 mg or more and less than 250 mg.

In the method of inhibiting TDP-43 aggregation or method of inhibiting cell death in TDP-43-overexpressing cells, a single dose of edaravone may be 30 mg or more and less than 60 mg.

In the method of inhibiting TDP-43 aggregation or method of inhibiting cell death in TDP-43-overexpressing cells, acamprosate may not be administered to a subject.

An aspect of the present invention provides an agent for treating or preventing diseases associated with TDP-43 aggregation, the agent including a combination of at least one selected from the group consisting of cycloserine, terizidone, and salts thereof, and edaravone.

In the agent for treating or preventing diseases associated with TDP-43 aggregation, the active component may essentially consist of a combination of at least one selected from the group consisting of cycloserine, terizidone, and salts thereof, and edaravone.

In the agent for treating or preventing diseases associated with TDP-43 aggregation, the active component may be a combination of at least one selected from the group consisting of cycloserine, terizidone, and salts thereof, and edaravone.

In the agent for treating or preventing diseases associated with TDP-43 aggregation, the at least one selected from the group consisting of cycloserine, terizidone, and salts thereof may be cycloserine or salt thereof.

In the agent for treating or preventing diseases associated with TDP-43 aggregation, the cycloserine may be D-cycloserine. In the agent for treating or preventing diseases associated with TDP-43 aggregation, the cycloserine may be L-cycloserine.

In the agent for treating or preventing diseases associated with TDP-43 aggregation, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

The agent for treating or preventing diseases associated with TDP-43 aggregation may include 15 mg or more and less than 250 mg of at least one selected from the group consisting of cycloserine, terizidone, and salts thereof.

The agent for treating or preventing diseases associated with TDP-43 aggregation may include 30 mg or more and 60 mg or less of edaravone.

The agent for treating or preventing diseases associated with TDP-43 aggregation may not include acamprosate.

An aspect of the present invention provides a combination of at least one selected from the group consisting of cycloserine, terizidone, and salts thereof, and edaravone, which is used to treat or prevent diseases associated with TDP-43 aggregation.

In the combination, the active component may essentially consist of a combination of at least one selected from the group consisting of cycloserine, terizidone, and salts thereof, and edaravone.

In the combination, the active component may be a combination of at least one selected from the group consisting of cycloserine, terizidone, and salts thereof, and edaravone.

In the combination, the at least one selected from the group consisting of cycloserine, terizidone, and salts thereof may be cycloserine or salt thereof.

In the combination, the cycloserine may be D-cycloserine. In the combination, the cycloserine may be L-cycloserine.

In the combination, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

The combination may include 15 mg or more and less than 250 mg of at least one selected from the group consisting of cycloserine, terizidone, and salts thereof.

The combination may include 30 mg or more and 60 mg or less of edaravone.

The combination may not include acamprosate.

An aspect of the present invention provides a use of a combination of at least one selected from the group consisting of cycloserine, terizidone, and salts thereof, and edaravone, which is used to produce an agent for treating or preventing diseases associated with TDP-43 aggregation.

In the use, the active component of the agent for treating or preventing diseases associated with TDP-43 aggregation may essentially consist of a combination of at least one selected from the group consisting of cycloserine, terizidone, and salts thereof, and edaravone.

In the use, the active component of the agent for treating or preventing diseases associated with TDP-43 aggregation may be a combination of at least one selected from the group consisting of cycloserine, terizidone, and salts thereof, and edaravone.

In the use, the at least one selected from the group consisting of cycloserine, terizidone, and salts thereof may be cycloserine or salt thereof.

In the use, the cycloserine may be D-cycloserine. In the use, the cycloserine may be L-cycloserine.

In the use, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

In the use, the combination may include 15 mg or more and less than 250 mg of at least one selected from the group consisting of cycloserine, terizidone, and salts thereof.

In the use, the combination may include 30 mg or more and 60 mg or less of edaravone.

In the use, the combination may not include acamprosate.

An aspect of the present invention provides a method of treating or preventing diseases associated with TDP-43 aggregation in a subject, the method including administering a combination of at least one selected from the group consisting of cycloserine, terizidone, and salts thereof, and edaravone to a subject.

In the method of treating or preventing diseases associated with TDP-43 aggregation, an active component that inhibits TDP-43 aggregation may essentially consist of a combination of at least one selected from the group consisting of cycloserine, terizidone, and salts thereof, and edaravone.

In the method of treating or preventing diseases associated with TDP-43 aggregation, the active component that inhibits TDP-43 aggregation may be a combination of at least one selected from the group consisting of cycloserine, terizidone, and salts thereof, and edaravone.

In the method of treating or preventing diseases associated with TDP-43 aggregation, the at least one selected from the group consisting of cycloserine, terizidone, and salts thereof may be cycloserine or salt thereof.

In the method of treating or preventing diseases associated with TDP-43 aggregation, the cycloserine may be D-cycloserine. In the method of treating or preventing diseases associated with TDP-43 aggregation, the cycloserine may be L-cycloserine.

In the method of treating or preventing diseases associated with TDP-43 aggregation, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

In the method of treating or preventing diseases associated with TDP-43 aggregation, a single dose of at least one selected from the group consisting of cycloserine, terizidone, and salts thereof may be 15 mg or more and less than 250 mg.

In the method of treating or preventing diseases associated with TDP-43 aggregation, a single dose of edaravone may be 30 mg or more and 60 mg or less.

In the method of treating or preventing diseases associated with TDP-43 aggregation, acamprosate may not be administered to a subject.

An aspect of the present invention provides an agent for treating or preventing diseases associated with TDP-43 aggregation, the agent including at least one selected from the group consisting of cycloserine, terizidone, and salts thereof, used in combination with edaravone.

In the agent for treating or preventing diseases associated with TDP-43 aggregation, the at least one selected from the group consisting of cycloserine, terizidone, and salts thereof may not be used in combination with an active component other than edaravone.

In the agent for treating or preventing diseases associated with TDP-43 aggregation, the at least one selected from the group consisting of cycloserine, terizidone, and salts thereof may be cycloserine or salt thereof.

In the agent for treating or preventing diseases associated with TDP-43 aggregation, the cycloserine may be D-cycloserine. In the agent for treating or preventing diseases associated with TDP-43 aggregation, the cycloserine may be L-cycloserine.

In the agent for treating or preventing diseases associated with TDP-43 aggregation, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

The agent for treating or preventing diseases associated with TDP-43 aggregation may include 15 mg or more and less than 250 mg of at least one selected from the group consisting of cycloserine, terizidone, and salts thereof.

In the agent for treating or preventing diseases associated with TDP-43 aggregation, the at least one selected from the group consisting of cycloserine, terizidone, and salts thereof may be used in combination with 30 mg or more and 60 mg or less of edaravone.

The agent for treating or preventing diseases associated with TDP-43 aggregation may not include acamprosate.

An aspect of the present invention provides at least one selected from the group consisting of cycloserine, terizidone, and salts thereof, used in combination with edaravone, which is used to treat or prevent diseases associated with TDP-43 aggregation.

The at least one selected from the group consisting of cycloserine, terizidone, and salts thereof may not be used in combination with an active component other than edaravone.

The at least one selected from the group consisting of cycloserine, terizidone, and salts thereof may be cycloserine or salt thereof.

In the at least one selected from the group consisting of cycloserine, terizidone, and salts thereof, the cycloserine may be D-cycloserine. In the at least one selected from the group consisting of cycloserine, terizidone, and salts thereof, the cycloserine may be L-cycloserine.

In the at least one selected from the group consisting of cycloserine, terizidone, and salts thereof, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

The mass of the at least one selected from the group consisting of cycloserine, terizidone, and salts thereof may be 15 mg or more and less than 250 mg.

The mass of edaravone used in combination

with the at least one selected from the group consisting of cycloserine, terizidone, and salts thereof may be 30 mg or more and less than 60 mg.

The at least one selected from the group consisting of cycloserine, terizidone, and salts thereof may not be used in combination with acamprosate.

An aspect of the present invention provides a use of at least one selected from the group consisting of cycloserine, terizidone, and salts thereof, used in combination with edaravone, which is used to produce an agent for treating or preventing diseases associated with TDP-43 aggregation.

In the use, the at least one selected from the group consisting of cycloserine, terizidone, and salts thereof may not be used in combination with an active component other than edaravone.

In the use, the at least one selected from the group consisting of cycloserine, terizidone, and salts thereof may be cycloserine or salt thereof.

In the use, the cycloserine may be D-cycloserine. In the use, the cycloserine may be L-cycloserine.

In the use, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

In the use, the mass of the at least one selected from the group consisting of cycloserine, terizidone, and salts thereof may be 15 mg or more and less than 250 mg.

In the use, the mass of edaravone used in combination with at least one selected from the group consisting of cycloserine, terizidone, and salts thereof may be 30 mg or more and less than 60 mg.

In the use, the at least one selected from the group consisting of cycloserine, terizidone, and salts thereof may not be used in combination with acamprosate.

An aspect of the present invention provides a method of treating or preventing diseases associated with TDP-43 aggregation in a subject, the method including administering at least one selected from the group consisting of cycloserine, terizidone, and salts thereof, used in combination with edaravone to a subject.

In the method of treating or preventing diseases associated with TDP-43 aggregation, the at least one selected from the group consisting of cycloserine, terizidone, and salts thereof may not be used in combination with an active component other than edaravone.

In the method of treating or preventing diseases associated with TDP-43 aggregation, the at least one selected from the group consisting of cycloserine, terizidone, and salts thereof may be cycloserine or salt thereof.

In the method of treating or preventing diseases associated with TDP-43 aggregation, the cycloserine may be D-cycloserine. In the method of treating or preventing diseases associated with TDP-43 aggregation, the cycloserine may be L-cycloserine.

In the method of treating or preventing diseases associated with TDP-43 aggregation, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

In the method of treating or preventing diseases associated with TDP-43 aggregation, a single dose of at least one selected from the group consisting of cycloserine, terizidone, and salts thereof may be 15 mg or more and less than 250 mg.

In the method of treating or preventing diseases associated with TDP-43 aggregation, the at least one selected from the group consisting of cycloserine, terizidone, and salts thereof may be used in combination with 30 mg or more and 60 mg or less of edaravone.

In the method of treating or preventing diseases associated with TDP-43 aggregation, acamprosate may not be administered to a subject.

An aspect of the present invention provides an agent for treating or preventing diseases associated with TDP-43 aggregation, the agent including edaravone, used in combination with at least one selected from the group consisting of cycloserine, terizidone, and salts thereof.

In the agent for treating or preventing diseases associated with TDP-43 aggregation, edaravone may not be used in combination with an active component other than at least one selected from the group consisting of cycloserine, terizidone, and salts thereof.

In the agent for treating or preventing diseases associated with TDP-43 aggregation, the at least one selected from the group consisting of cycloserine, terizidone, and salts thereof may be cycloserine or salt thereof.

In the agent for treating or preventing diseases associated with TDP-43 aggregation, the cycloserine may be D-cycloserine. In the agent for treating or preventing diseases associated with TDP-43 aggregation, the cycloserine may be L-cycloserine.

In the agent for treating or preventing diseases associated with TDP-43 aggregation, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

In the agent for treating or preventing diseases associated with TDP-43 aggregation, edaravone may be used in combination with 15 mg or more and less than 250 mg of at least one selected from the group consisting of

cycloserine, terizidone, and salts thereof.

The agent for treating or preventing diseases associated with TDP-43 aggregation may include 30 mg or more and 60 mg or less of edaravone.

The agent for treating or preventing diseases associated with TDP-43 aggregation may not include acamprosate.

An aspect of the present invention provides edaravone used in combination with at least one selected from the group consisting of cycloserine, terizidone, and salts thereof, which is used to treat or prevent diseases associated with TDP-43 aggregation.

The edaravone may not be used in combination with an active component other than at least one selected from the group consisting of cycloserine, terizidone, and salts thereof.

The at least one selected from the group consisting of cycloserine, terizidone, and salts thereof used in combination with edaravone may be cycloserine or salt thereof.

The cycloserine used in combination with edaravone may be D-cycloserine. The cycloserine used in combination with edaravone may be L-cycloserine.

In edaravone, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

The edaravone may be used in combination with 15 mg or more and less than 250 mg of at least one selected from the group consisting of cycloserine, terizidone, and salts thereof.

The mass of edaravone may be 30 mg or more and 60 mg or less.

The edaravone may not be used in combination with acamprosate.

An aspect of the present invention provides a use of edaravone used in combination with at least one selected from the group consisting of cycloserine, terizidone, and salts thereof, which is used to produce an agent for treating or preventing diseases associated with TDP-43 aggregation.

In the use, edaravone may not be used in combination with an active component other than at least one selected from the group consisting of cycloserine, terizidone, and salt thereof.

In the use, the at least one selected from the group consisting of cycloserine, terizidone, and salts thereof may be cycloserine or salts thereof.

In the use, the cycloserine may be D-cycloserine. In the use, the cycloserine may be L-cycloserine.

In the use, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

In the use, the mass of the at least one selected from the group consisting of cycloserine, terizidone, and salts thereof used in combination with edaravone may be 15 mg or more and less than 250 mg.

In the use, the mass of edaravone may be 30 mg or more and less than 60 mg.

In the use, edaravone may not be used in combination with acamprosate.

An aspect of the present invention provides a method of treating or preventing diseases associated with TDP-43 aggregation in a subject, the method including administering edaravone to a subject, used in combination with at least one selected from the group consisting of cycloserine, terizidone, and salts thereof.

In the method of treating or preventing diseases associated with TDP-43 aggregation, edaravone may not be used in combination with an active component other than at least one selected from the group consisting of cycloserine, terizidone, and salts thereof.

In the method of treating or preventing diseases associated with TDP-43 aggregation, the at least one selected from the group consisting of cycloserine, terizidone, and salts thereof may be cycloserine or salt thereof.

In the method of treating or preventing diseases associated with TDP-43 aggregation, the cycloserine may be D-cycloserine. In the method of treating or preventing diseases associated with TDP-43 aggregation, the cycloserine may be L-cycloserine.

In the method of treating or preventing diseases associated with TDP-43 aggregation, the disease associated with TDP-43 aggregation may be TDP-43 proteinopathy. The TDP-43 proteinopathy may be at least one selected from the group consisting of amyotrophic lateral sclerosis, frontotemporal lobar degeneration, primary lateral sclerosis, and progressive muscular atrophy. The TDP-43 proteinopathy may be amyotrophic lateral sclerosis.

In the method of treating or preventing diseases associated with TDP-43 aggregation, a single dose of at least one selected from the group consisting of cycloserine, terizidone, and salts thereof used in combination with edaravone may be 15 mg or more and less than 250 mg.

In the method of treating or preventing diseases associated with TDP-43 aggregation, a single dose of edaravone may be 30 mg or more and less than 60 mg.

In the method of treating or preventing diseases associated with TDP-43 aggregation, acamprosate may not be administered to a subject.

## Advantageous Effect of Invention

According to the present invention, it is possible to provide a suppressor of TDP-43 aggregation, a suppressor of cell death in TDP-43-overexpressing cells,

and an agent for treating or preventing diseases associated with TDP-43 aggregation.

**Brief Description of Drawings**

[Figure 1] Figure 1 is an image showing soluble fractions of TDP-43 and insoluble fractions of TDP-43 in Reference Example 1.

[Figure 2] Figure 2 is a graph showing the relationship between the elapsed time from transfection of the TDP-43 gene in Reference Example 1, and the proportion of insoluble fractions of TDP-43 in cells.

[Figure 3] Figure 3 shows images of cells in Reference Example 2.

[Figure 4] Figure 4 is a graph showing the relationship between the concentration of a compound in Reference Example 2 and the relative cell viability.

[Figure 5] Figure 5 is an image showing soluble fractions of TDP-43 and insoluble fractions of TDP-43 in Reference Example 3.

[Figure 6] Figure 6 is a graph showing the relationship between a compound in Reference Example 3 and the proportion of insoluble fractions of TDP-43 in cells.

[Figure 7] Figure 7 is an image showing soluble fractions of TDP-43 and insoluble fractions of TDP-43 in Reference Example 4.

[Figure 8] Figure 8 is a graph showing the proportion of insoluble fractions of TDP-43 in Reference Example 4.

[Figure 9] Figure 9 is a graph showing the relationship between the concentration of a compound in Example 1 and the relative cell viability.

[Figure 10] Figure 10 is a graph showing $EC_{50}$ of the cell death suppressory effect of D-cycloserine at each concentration of edaravone in Example 1.

[Figure 11] Figure 11 is a table showing $EC_{50}$ of the cell death suppressory effect of D-cycloserine at each concentration of edaravone in Example 1, and combination index values.

**Description of Embodiments**

Embodiments of the present invention will be described below. However, it should not be understood that the following embodiments limit the present invention. Those skilled in the art can clearly understand various alternative embodiments, examples and operational techniques from the present disclosure. It should be understood that the present invention includes various embodiments and the like that are not described herein.

An agent for treating or preventing diseases associated with TAR DNA-binding protein of 43kDa (TDP-43) aggregation, a suppressor of TDP-43 aggregation, or a suppressor of cell death in TDP-43-overexpressing cells according to an embodiment includes a combination of at least one selected from the group consisting of cycloserine and physiologically acceptable salts thereof, and edaravone.

In addition, the agent for treating or preventing diseases associated with TDP-43 aggregation, the suppressor of TDP-43 aggregation, or the suppressor of cell death in TDP-43-overexpressing cells according to the embodiment includes at least one selected from the group consisting of cycloserine and physiologically acceptable salts thereof used in combination with edaravone.

In addition, the agent for treating or preventing diseases associated with TDP-43 aggregation, the suppressor of TDP-43 aggregation, or the suppressor of cell death in TDP-43-overexpressing cells according to the embodiment includes edaravone used in combination with at least one selected from the group consisting of cycloserine and physiologically acceptable salts thereof.

Examples of diseases associated with TDP-43 aggregation include TDP-43 proteinopathy, neurodegenerative diseases, and muscular diseases.

Examples of TDP-43 proteinopathies include amyotrophic lateral sclerosis (ALS), frontotemporal lobar degeneration (FTLD), primary lateral sclerosis (PLS), and progressive muscular atrophy (PMA). The frontotemporal lobar degeneration (FTLD) may be frontotemporal lobar degeneration (FTLD-TDP) associated with TDP-43 accumulation.

Examples of neurodegenerative diseases include dementia with Lewy bodies (DLB), corticobasal degeneration (CBD), progressive supranuclear palsy (PSP), Guam-type ALS/Parkinsonism-dementia complex, hippocampal sclerosis, Pick's disease, Alzheimer's disease, Huntington's disease, Parkinson's disease, argyrophilic grain disease (AGD), chronic traumatic encephalopathy (CTE), multiple system atrophy (MSA), and limbic-predominant age-related TDP-43 encephalopathy.

Examples of muscular diseases include inclusion body myopathy with Paget's disease of bone and frontotemporal dementia, sporadic IBM, myofibrillar myopathy, oculopharyngeal muscular dystrophy, and distal myopathy with rimmed vacuoles.

The cycloserine may be D-cycloserine or L-cycloserine.

The IUPAC name for D-cycloserine (CAS number: 68-41-7) is (4R)-4-Amino-1,2-oxazolidin-3-one. The chemical formula of D-cycloserine is $C_3H_6N_2O_2$. The chemical structural formula of D-cycloserine is as follows.

[Chem. 1]

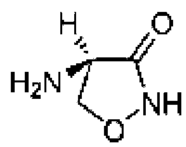

The IUPAC name for L-cycloserine (CAS number: 339-72-0) is (4S)-4-amino-1,2-oxazolidin-3-one. The chemical formula of L-cycloserine is $C_3H_6N_2O_2$. The chemical structural formula of L-cycloserine is as follows.

[Chem. 2]

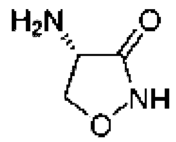

The IUPAC name for edaravone (CAS number: 89-25-8) is 5-methyl-2-phenyl-2,4-dihydro-3H-pyrazol-3-one. The chemical formula of edaravone is $C_{10}H_{10}N_2O$. The chemical structural formula of edaravone is as follows.

[Chem. 3]

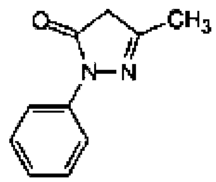

The agent for treating or preventing diseases associated with TDP-43 aggregation, the suppressor of TDP-43 aggregation, or the suppressor of cell death in TDP-43-overexpressing cells according to the embodiment may include at least one prodrug of the compound or physiologically acceptable salts thereof.

For example, terizidone is known as a prodrug of D-cycloserine. The IUPAC name for terizidone (CAS number: 25683-71-0) is 4,4'-{1,4-Phenylenebis[(E)methylylidene(E)azanylylidene]}bis(1,2-oxazolidin-3-one). The chemical formula of terizidone is $C_{14}H_{14}N_4O_4$. The chemical structural formula of terizidone is as follows. Terizidone is decomposed in the body and exhibits effects similar to D-cycloserine.

[Chem. 4]

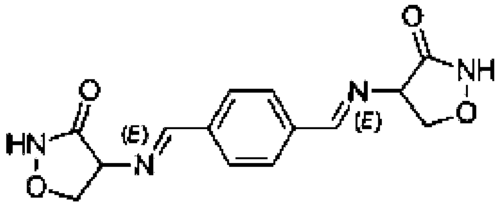

The agent for treating or preventing diseases associated with TDP-43 aggregation, the suppressor of TDP-43 aggregation, or the suppressor of cell death in TDP-43-overexpressing cells according to the embodiment can be formulated into a pharmaceutically acceptable dosage form. For example, the agent for treating or preventing diseases associated with TDP-43 aggregation, the suppressor of TDP-43 aggregation, or the suppressor of cell death in TDP-43-overexpressing cells can be formulated into an injectable agent, a solution, a suspension, a tablet, a capsule, a pill, a granule, a syrup, a suppository, an inhalant, or a spray. Examples of injectable agents include an injectable solution, a sterile powder for injection, and a concentrated solution for injection.

It has been reported that, when 15 mg of D-cycloserine is orally administered to humans, the maximum blood concentration (Cmax) of D-cycloserine is about 5.6 μmol/L (van Berckel, B., Lipsch, C., Timp, S. et al. Behavioral and Neuroendocrine Effects of the Partial NMDA Agonist D-cycloserine in Healthy Subjects. Neuropsychopharmacol 16, 317-324 (1997). https://doi.org/10.1016/S0893-133X(96)00196-0, and van Berckel, B. Erratum: Behavioral and Neuroendocrine Effects of the Partial NMDA Agonist D-cycloserine in Healthy Subjects. Neuropsychopharmacol 17, 116 (1997). https://doi.org/10.1016/S0893-133X(97)00082-1).

The brain penetration rate of D-cycloserine has been reported to be 95%. Therefore, the maximum concentration of D-cycloserine in the brain is estimated to be about 5.3 μmol/L. The concentration of about 5.3 μmol/L exceeds an $EC_{50}$ of 128 nmol/L of D-cycloserine, which inhibits cell death in nerve cells forcibly expressing TDP-43, as shown in Reference Example 2 to be described below. In addition, the half-life of D-cycloserine has been reported to be about 10 hours. Therefore, it is estimated that the trough value when 15 mg of D-cycloserine is orally administered twice daily exceeds an $EC_{50}$ of 128 nmol/L. Therefore, when the agent for treating or preventing diseases associated with TDP-43 aggregation, the suppressor of TDP-43 aggregation, or the suppressor of cell death in TDP-43-overexpressing cells according to the embodiment includes 15 mg or more of at least one selected from the group consisting of cycloserine, terizidone, and salts thereof, it is possible to sufficiently exhibit the effect of treating or preventing diseases associated with TDP-43 aggregation such as human amyotrophic lateral sclerosis.

In addition, it has been reported that, when 250 mg of D-cycloserine is administered to humans in order to treat tuberculosis, it causes serious side effects such as epileptiform seizure and mental confusion. Therefore, when the agent for treating or preventing diseases associated with TDP-43 aggregation, the suppressor of TDP-43 aggregation, or the suppressor of cell death in TDP-43-overexpressing cells according to the embodiment includes less than 250 mg of at least one selected from the group consisting of cycloserine, terizidone, and salts thereof, it is possible to inhibit side effects caused by at least one selected from the group consisting of cycloserine, terizidone, and salts thereof.

It should be noted that, it has been reported that, when 150 mg of D-cycloserine is administered to humans, it causes headaches. However, since diseases associated with TDP-43 aggregation such as amyotrophic lateral sclerosis are extremely serious diseases, even if headaches occur as a side effect, treatment should be prioritized for such diseases.

The agent for treating or preventing diseases associated with TDP-43 aggregation, the suppressor of TDP-43 aggregation, or the suppressor of cell death in TDP-43-overexpressing cells according to the embodiment may include, for example, 17 mg or more, 20 mg or more, or 25 mg or more of at least one selected from the group consisting of cycloserine, terizidone, and salts thereof.

The agent for treating or preventing diseases associated with TDP-43 aggregation, the suppressor of TDP-43 aggregation, or the suppressor of cell death in TDP-43-overexpressing cells according to the embodiment may include, for example, 249.9 mg or less, 249 mg or less, 240 mg or less, 230 mg or less, 220 mg or less, 210 mg or less, 200 mg or less, 190 mg or less, 180 mg or less, 170 mg or less, 160 mg or less, 150 mg or less, 100 mg or less, 80 mg or less, or 60 mg or less of at least one selected from the group consisting of cycloserine, terizidone, and salts thereof.

When the agent for treating or preventing diseases associated with TDP-43 aggregation, the suppressor of TDP-43 aggregation, or the suppressor of cell death in TDP-43-overexpressing cells according to the embodiment includes 15 mg or more and less than 250 mg of at least one selected from the group consisting of cycloserine, terizidone, and salts thereof, the administered at least one selected from the group consisting of cycloserine and salts thereof acts in an agonist-like manner on NMDA receptors.

The agent for treating or preventing diseases associated with TDP-43 aggregation, the suppressor of TDP-43 aggregation, or the suppressor of cell death in TDP-43-overexpressing cells according to the embodiment includes, for example, 30 mg or more and 60 mg or less of edaravone. When the agent for treating or preventing diseases associated with TDP-43 aggregation, the suppressor of TDP-43 aggregation, or the suppressor of cell death in TDP-43-overexpressing cells according to the embodiment includes 30 mg or more and 60 mg or less of edaravone, it is possible to inhibit side effects of edaravone and exhibit efficacy of edaravone. Examples of side effects of edaravone include liver dysfunction and rash. The agent for treating or preventing diseases associated with TDP-43 aggregation, the suppressor of TDP-43 aggregation, or the suppressor of cell death in TDP-43-overexpressing cells according to the embodiment may include, for example, 32 mg or more, 34 mg or more, or 36 mg or more of edaravone. The agent for treating or preventing diseases associated with TDP-43 aggregation, the suppressor of TDP-43 aggregation, or the suppressor of cell death in TDP-43-overexpressing cells according to the embodiment may include, for example, 58 mg or less, 56 mg or less, or 54 mg or less of edaravone.

The agent for treating or preventing diseases associated with TDP-43 aggregation, the suppressor of TDP-43 aggregation, or the suppressor of cell death in TDP-43-overexpressing cells according to the embodiment may include a mixture of at least one selected from the group consisting of cycloserine and physiologically acceptable salts thereof, and edaravone. At least one selected from the group consisting of cycloserine and physiologically acceptable salts thereof, and edaravone may be formulated together. Alternatively, in the agent for treating or preventing diseases associated with TDP-43 aggregation, the suppressor of TDP-43 aggregation, or the suppressor of cell death in TDP-43-overexpressing cells according to the embodiment, at least one selected from the group consisting of cycloserine and physiologically acceptable salts thereof, and edaravone may be formulated separately.

At least one selected from the group consisting of cycloserine and physiologically acceptable salts thereof, and edaravone can be used in combination therapy. At least one selected from the group consisting of cycloserine and physiologically acceptable salts thereof, and edaravone may be administered to a subject simultaneously or at different times. At least one selected from the group consisting of cycloserine and physiologically acceptable salts thereof may be administered to a subject first, and edaravone may be administered to the subject several minutes, several hours, or several days thereafter. Edaravone may be administered to a subject first and at least one selected from the group consisting of cycloserine and physiologically acceptable salts thereof may be administered to the subject several minutes, several hours, or several days thereafter.

As described above, it is strongly suggested that TDP-43 aggregation causes abnormalities in RNA metabolism and induces cytotoxicity, leading to the onset and progression of various diseases. The agent for treating or preventing diseases associated with TDP-43 aggregation according to the embodiment can treat or prevent diseases associated with TDP-43 aggregation by inhibiting TDP-43 aggregation. Alternatively, the agent for treating or preventing diseases associated with TDP-43 aggregation according to the embodiment can treat or prevent diseases associated with TDP-43 aggregation by inhibiting cell death in TDP-43-overexpressing cells,.

The agent for treating or preventing diseases associated with TDP-43 aggregation, the suppressor of TDP-43 aggregation, or the suppressor of cell death in TDP-43-overexpressing cells according to the embodiment may not include acamprosate. Acamprosate is a derivative of homotaurine, and also known as 3-(acetylamino)propylsulfonic acid or N-acetylhomotaurine. When acamprosate is administered to humans, it can

cause side effects such as anaphylaxis associated with symptoms such as generalized skin rash, rash, urticaria, stomatitis, laryngospasm, and shortness of breath. In addition, when acamprosate is administered to humans, it can cause side effects such as angioedema associated with symptoms such as tongue swelling and lymphadenopathy. Since the agent for treating or preventing diseases associated with TDP-43 aggregation, the suppressor of TDP-43 aggregation, or the suppressor of cell death in TDP-43-overexpressing cells according to the embodiment does not need to be combined with acamprosate, these side effects do not occur.

While the present invention has been described above with reference to the embodiments, the descriptions and drawings that form some of the disclosure should not be understood as limiting the invention. Those skilled in the art can clearly understand various alternative embodiments, examples and operational techniques from this disclosure. It should be understood that the present invention includes various embodiments and the like that are not described herein.

(Reference Example 1)

Mouse neuroblastoma (Neuro2a) cells were seeded on a dish. The Neuro2a cells were cultured in a culture medium containing DMEM+10% FBS at 37°C in the presence of 5% $CO_2$. On the first day after seeding, differentiation into neurons was initiated. Differentiation into neurons was performed in a culture medium containing DMEM+2% FBS+20 μmol/L retinoic acid. On the fourth day after seeding, cells were transfected with synthesized TDP-43 mRNA by a lipofection method. Cells were sampled at 6 hours, 9 hours, 12 hours, 15 hours, 18 hours, and 21 hours after transfection. At 21 hours after transfection, cell death was observed. For sampling, cells were lysed in an RIPA buffer and centrifuged at 22,000×g for 30 minutes at 4°C, the supernatant was used as the soluble fraction, the precipitate was used as the insoluble fraction, and equal volumes of a 2×SDS sample buffer were added to the respective fractions and heated at 95°C for 5 minutes to prepare electrophoresis samples. The electrophoresis samples were subjected to electrophoresis using a polyacrylamide gel, TDP-43 was then transferred to a PVDF membrane. TDP-43 was detected through chemiluminescence using anti-TDP-43 antibodies as primary antibodies and HRP-conjugated anti-rabbit antibodies as secondary antibodies. These results are shown in Figure 1 and Figure 2. It was confirmed that the number of insoluble fractions of TDP-43 increased in the cells between the transfection of the TDP-43 gene and the onset of cell death. The insoluble fractions of TDP-43 indicate the aggregation of TDP-43.

(Reference Example 2)

Neuro2a cells were seeded on a dish. On the first day after seeding, differentiation into neurons was initiated. On the fourth day after seeding, cells were transfected with the TDP-43 gene. At 5 hours after transfection, 10 nmol/L, 50 nmol/L, 100 nmol/L, 500 nmol/L, 1,000 nmol/L, 5,000 nmol/L, or 10,000 nmol/L of D-cycloserine or L-cycloserine was added to a culture medium. In addition, as a control, 10 nmol/L, 50 nmol/L, 100 nmol/L, 500 nmol/L, 1,000 nmol/L, 5,000 nmol/L, or 10,000 nmol/L of ropinirole was added to a culture medium. Ropinirole has been reported to have an effect of treating ALS.

The proportion of dead cells was evaluated 7 days after the cells were transfected with the TDP-43 gene. As shown in Figure 3A, no cell death was observed in cells which were not transfected with the TDP-43 gene. As shown in Figure 3B, cell death was observed in cells which were transfected with the TDP-43 gene and not supplemented with D-cycloserine, L-cycloserine, or ropinirole. As shown in Figure 3C, cell death was inhibited in cells which were transfected with the TDP-43 gene and supplemented with D-cycloserine.

As shown in Figure 4, D-cycloserine and L-cycloserine exhibited an effect of inhibiting cell death in a concentration-dependent manner. The $EC_{50}$ of D-cycloserine was 128 nmol/L. The $EC_{50}$ of L-cycloserine was 237 nmol/L. The $EC_{50}$ of ropinirole was 198 nmol/L. Here, the relative viability indicates the proportion of the number of viable cells overexpressing TDP-43 based on 100% of the number of viable cells not overexpressing TDP-43.

(Reference Example 3)

Neuro2a cells were seeded on a dish. On the first day after seeding, differentiation into neurons was initiated. On the fourth day after seeding, cells were transfected with the TDP-43 gene. At 6 hours after transfection, 10 μmol/L of D-cycloserine, L-cycloserine, ropinirole, or DMSO was added to the cells, and the cells were cultured for 12 hours. Then, the results obtained by analyzing the soluble fractions and insoluble fractions of TDP-43 in the cells are shown in Figure 5. As shown in Figure 5, as a control, in cells treated with DMSO, it was confirmed that the number of insoluble fractions of TDP-43 was larger than the number of soluble fractions of TDP-43. On the other hand, in cells treated with D-cycloserine and L-cycloserine, it was confirmed that the number of soluble fractions of TDP-43 was larger than the number of insoluble fractions of TDP-43.

The results obtained by quantifying the proportion of insoluble fractions based on a total number of soluble fractions and insoluble fractions of TDP-43 in the cells are shown in Figure 6. In cells treated with D-cycloserine and L-cycloserine, the proportion of insoluble fractions was less than half.

(Reference Example 4)

100 μL of an EHS-gel basement membrane matrix (commercially available from FUJIFILM Wako Pure Chemical Corporation) was added to 12.5 mL of DMEM (Dulbecco's Modified Eagle Medium, commercially available from FUJIFILM Wako Pure Chemical Corporation) and mixed, and the mixture was added to a 24-well plate at 600 μL per well. The plate was left at room temperature for 2 hours, and the solution was then removed from the wells. Next, 600 μL of DMEM was added to each well, and the plate was left at room temperature for 2 hours.

Motor neurons derived from iPS cells of healthy subjects and motor neurons derived from iPS cells of ALS patients were obtained from iXCells Biotechnologies. The motor neurons were quickly thawed in a thermostatic oven at 37°C and suspended in a motor neuron maintenance culture medium (iXCells Biotechnologies). The culture medium was centrifuged at 600 xg for 5 minutes to collect motor neurons, the culture medium was then removed, and the motor neurons were suspended in a motor neuron maintenance culture medium.

After motor neurons were diluted in a culture medium to a density of $3X10^5$ cells/mL, 600 μL of the culture medium containing motor neurons was added to each of a plurality of wells of a matrix-coated plate, and the motor neurons were cultured at 37°C in the presence of 5% $CO^2$. Every 2 or 3 days, 300 μL of the culture medium was removed from each of the plurality of wells, and 300 μL of a new culture medium was added to each of the plurality of wells.

On the seventh day after culture initiation, 300 μL of the culture medium was removed from each of the plurality of wells, and 300 μL of a culture medium containing 0.2% DMSO, a culture medium containing 20 μmol/L of D-cycloserine (Cayman Chemical), or a culture medium containing 20 μmol/L of ropinirole (commercially available from FUJIFILM Wako Pure Chemical Corporation) was added to each of the plurality of wells. Every 2 or 3 days, 300 μL of the culture medium was removed from each of the plurality of wells, 300 μL of one containing the same compound as the removed culture medium, that is, a culture medium containing 0.1 % DMSO, a culture medium containing 10 μmol/L of D-cycloserine, or a culture medium containing 10 μmol/L of ropinirole, was added to each of the plurality of wells.

5 μL of a protease suppressor and phosphatase suppressor cocktail (Halt Protease and Phosphatase Suppressor Cocktail, 10OX, registered trademark, Thermo Fisher Scientific) was added to 500 μL of a protein extraction buffer solution containing a surfactant (RIPA buffer, commercially available from Nacalai Tesque, Inc.) to prepare a cell extraction solution. 50 μL of the cell extraction solution was added to the motor neurons cultured for 20 days in the presence of DMSO, D-cycloserine, or ropinirole, and the cell extraction solution was collected in a 1.5 mL tube by pipetting. The cell extraction solution was centrifuged at 4°C, 21,000 Xg for 30 minutes.

After centrifugation, 45 μL of the supernatant was collected, 45 μL of an electrophoresis buffer solution (AE-1430 EzApply, Atto) was then added to the supernatant and heated at 95°C for 5 minutes to prepare soluble protein fractions. In addition, after centrifugation, the precipitate was washed twice with 50 μL of an RIPA buffer, 45 μL of the RIPA buffer and 45 μL of the electrophoresis buffer solution were then added to the precipitate and heated at 95°C for 5 minutes to prepare insoluble protein fractions.

The soluble protein fractions and the insoluble protein fractions were subjected to SDS-PAGE, and the proteins were transferred to a PVDF membrane. The PVDF membrane was immersed in a blocking solution and shaken at room temperature for 1 hour to block the PVDF membrane. The PVDF membrane was immersed in solution containing TDP-43 polyclonal antibodies (PGI Proteintech Group) and shaken at 4°C overnight. Then, the PVDF membrane was washed three times with a buffer solution, and the PVDF membrane was then immersed in a solution containing HRP (horseradish peroxidase)-labeled secondary antibodies (Anti-Rabbit IgG, HRP-Linked F(ab')2 Fragment Donkey, Cytiva) and shaken at room temperature for 1 hour. Next, the PVDF membrane was washed three times with a buffer solution, 1 mL of a luminescence reagent (ImmunoStar LD, commercially available from FUJIFILM Wako Pure Chemical Corporation) was then used to induce luminescence from the labeled secondary antibodies, and the PVDF membrane was imaged. The imaged PVDF membrane is shown in Figure 7.

As shown in Figure 7, as a control, in motor neurons derived from iPS cells of healthy subjects cultured in the presence of DMSO, bands for the soluble fractions of TDP-43 were clearly observed, and bands for insoluble fractions were unclear. In motor neurons derived from iPS cells of healthy subjects cultured in the presence of D-cycloserine, bands for soluble fractions of TDP-43 were clearly observed, and bands for insoluble fractions were unclear. In motor neurons derived from iPS cells of healthy subjects cultured in the presence of ropinirole, bands for soluble fractions of TDP-43 were clearly observed, and bands for insoluble fractions were unclear.

As shown in Figure 7, as a control, in motor neurons derived from iPS cells of ALS patients cultured in the presence of DMSO, bands for soluble fractions of TDP-43 were clearly observed, and bands for insoluble fractions were also clearly confirmed. In motor neurons derived from iPS cells of ALS patients cultured in the presence of D-cycloserine, bands for soluble fractions of TDP-43 were clearly observed, and bands for insoluble fractions were unclear. In motor neurons derived from iPS cells of ALS patients cultured in the presence of ropinirole, bands for soluble fractions of TDP-43 were clearly observed, and bands for insoluble fractions were

unclear.

The captured PVDF membrane image was opened in ImageJ software and the Invert command was used to invert black and white. After the background was removed using the Subtract Background command, the TDP-43 bands in the lanes were sequentially enclosed in rectangular frames of the same size, and the band intensity was quantified using the Measure command. The proportion of insoluble fractions of TDP-43 was calculated from the band intensity of soluble fractions of TDP-43 and the band intensity of insoluble fractions of TDP-43 for each sample according to the following formula.

$$R=\{I_I/(I_I+I_S)\}\times 100$$

In the formula, R is the proportion (%) of insoluble fractions of TDP-43, $I_I$ is the band intensity of insoluble fractions of TDP-43, and $I_S$ is the band intensity of soluble fractions of TDP-43.

Three experiments were performed, and the average proportion of insoluble fractions of TDP-43 was calculated. In Figure 8, the proportion of insoluble fractions of TDP-43 in each experiment is shown as a dot, and the average value is shown as a bar. In motor neurons derived from iPS cells of healthy subjects, the average proportion of insoluble fractions of TDP-43 was about 7.4%. On the other hand, in motor neurons derived from iPS cells of ALS patients, the average proportion of insoluble fractions of TDP-43 was about 46.1%. However, in motor neurons derived from iPS cells of ALS patients in the presence of D-cycloserine, the average proportion of insoluble fractions of TDP-43 decreased to about 17.4%. In addition, in motor neurons derived from iPS cells of ALS patients in the presence of ropinirole, the average proportion of insoluble fractions of TDP-43 decreased to about 19.3%.

FTLD-TDP and ALS have many common features, including accumulation of TDP-43. Therefore, the decrease in the average proportion of insoluble fractions of TDP-43 in motor neurons derived from iPS cells of ALS patients in the presence of D-cycloserine indicates that administration of D-cycloserine can inhibit TDP-43 accumulation in cells of FTLD-TDP patients.

(Example 1)

Neuro2a cells were seeded on a dish. On the first day after seeding, differentiation into neurons was initiated. On the fourth day after seeding, cells were transfected with the TDP-43 gene. At 5 hours after transfection, 10 nmol/L, 30 nmol/L, 100 nmol/L, 300 nmol/L, 1,000 nmol/L, 3,000 nmol/L, or 10,000 nmol/L of D-cycloserine was added to a culture medium. Immediately thereafter, 10 nmol/L, 30 nmol/L, 100 nmol/L, 300 nmol/L, 1,000 nmol/L, 3,000 nmol/L, or 10,000 nmol/L of edaravone was additionally added to the culture medium containing D-cycloserine at each concentration.

The proportion of dead cells was evaluated 7 days after the cells were transfected with the TDP-43 gene. Figure 9 shows the relative cell viability at each concentration of D-cycloserine and edaravone. Here, the relative viability indicates the proportion of the number of viable cells overexpressing TDP-43 based on 100% of the number of viable cells not overexpressing TDP-43. In addition, Figure 10 and Figure 11 show the $EC_{50}$ of the cell death suppressory effect of D-cycloserine at each concentration of edaravone calculated from the results of Figure 9. When treated with edaravone at each concentration, the $EC_{50}$ of the cell death suppressory effect of D-cycloserine was 151.36 nmol/L at 0 nmol/L of edaravone, 149.44 nmol/L at 10 nmol/L of edaravone, 135.18 nmol/L at 30 nmol/L of edaravone, 118.85 nmol/L at 100 nmol/L of edaravone, 106.76 nmol/L at 300 nmol/L of edaravone, and 87.62 nmol/L at 1,000 nmol/L of edaravone.

Whether the use of D-cycloserine and edaravone in combination exhibited a synergistic effect was quantified using the combination index (CI) (Chou et al.) (refer to T C Chou, P Talalay., Adv Enzyme Regul. 1984;22: 27-55.). The CI was calculated using the following formula.

[Math. 1]

$$CI = \frac{D\,a}{D\,x,a} + \frac{D\,b}{D\,x,b}$$

s

In the formula, Da is the concentration of D-cycloserine at which the 50% cell death suppressory effect was exhibited when D-cycloserine and edaravone were used in combination, Db is the concentration of edaravone at which the 50% cell death suppressory effect was exhibited when D-cycloserine and edaravone were used in combination, Dxa is the concentration of D-cycloserine at which the 50% cell death suppressory effect was exhibited when D-cycloserine was used alone, and Dxb is the concentration of edaravone at which the 50% cell death suppressory effect was exhibited when edaravone was used alone.

CI=1 indicates an additive effect, CI>1 indicates an antagonistic effect, and CI<1 indicates a synergistic effect. As shown in Figure 11, when D-cycloserine and edaravone were used in combination, the CI was 0.99 at 10 nmol/L of edaravone, 0.91 at 30 nmol/L of edaravone, 0.83 at 100 nmol/L of edaravone, and 0.83 at 300 nmol/L of edaravone. This indicates that the use of D-cycloserine and edaravone in combination exhibited a synergistic effect.

**Claims**

1. A suppressor of TDP-43 aggregation, the suppressor comprising a combination of at least one selected from the group consisting of cycloserine, terizidone, and salts thereof, and edaravone.

2. The suppressor of TDP-43 aggregation according to claim 1, comprising cycloserine or salt thereof.

3. The suppressor of TDP-43 aggregation according to claim 1, wherein the cycloserine is D-cycloserine.

4. The suppressor of TDP-43 aggregation according to claim 1, wherein the cycloserine is L-cycloserine.

5. A suppressor of TDP-43 aggregation, the suppressor comprising at least one selected from the group consisting of cycloserine, terizidone, and salts thereof, used in combination with edaravone.

6. The suppressor of TDP-43 aggregation according to claim 5, comprising cycloserine or salt thereof.

7. The suppressor of TDP-43 aggregation according to claim 5, wherein the cycloserine is D-cycloserine.

8. The suppressor of TDP-43 aggregation according to claim 5, wherein the cycloserine is L-cycloserine.

9. The suppressor of TDP-43 aggregation according to claim 1, comprising 15 mg or more and less than 250 mg of at least one selected from the group consisting of cycloserine, terizidone, and salts thereof.

10. The suppressor of TDP-43 aggregation according to claim 1, wherein a content of the edaravone is 30 mg or more and 60 mg or less.

11. The suppressor of TDP-43 aggregation according to claim 1, which does not comprise acamprosate.

12. A suppressor of TDP-43 aggregation, the suppressor comprising edaravone used in combination with at least one selected from the group consisting of cycloserine, terizidone, and salts thereof.

13. The suppressor of TDP-43 aggregation according to claim 12, comprising cycloserine or salt thereof.

14. The suppressor of TDP-43 aggregation according to claim 12, wherein the cycloserine is D-cycloserine.

15. The suppressor of TDP-43 aggregation according to claim 12, wherein the cycloserine is L-cycloserine.

16. The suppressor of TDP-43 aggregation according to claim 12, which is used in combination with 15 mg or more and less than 250 mg of at least one selected from the group consisting of cycloserine, terizidone, and salts thereof.

17. The suppressor of TDP-43 aggregation according to claim 12, wherein a content of the edaravone is 30 mg or more and 60 mg or less.

18. The suppressor of TDP-43 aggregation according to claim 12, which does not comprise acamprosate.

19. A suppressor of cell death in TDP-43-overexpressing cells, the suppressor comprising a combination of at least one selected from the group consisting of cycloserine, terizidone, and salts thereof, and edaravone.

20. The suppressor of cell death in TDP-43-overexpressing cells according to claim 19, comprising cycloserine or salt thereof.

21. The suppressor of cell death in TDP-43-overexpressing cells according to claim 19, wherein the cycloserine is D-cycloserine.

22. The suppressor of cell death in TDP-43-overexpressing cells according to claim 19, wherein the cycloserine is L-cycloserine.

23. The suppressor of cell death in TDP-43-overexpressing cells according to claim 19, comprising 15 mg or more and less than 250 mg of at least one selected from the group consisting of cycloserine, terizidone, and salts thereof.

24. The suppressor of cell death in TDP-43-overexpressing cells according to claim 19, wherein a content of the edaravone is 30 mg or more and 60 mg or less.

25. The suppressor of cell death in TDP-43-overexpressing cells according to claim 19, which does not comprise acamprosate.

26. A suppressor of cell death in TDP-43-overexpressing cells, the suppressor comprising at least one selected from the group consisting of cycloserine, terizidone, and salts thereof, used in combination with edaravone.

27. The suppressor of cell death in TDP-43-overexpressing cells according to claim 26, comprising cycloserine or salt thereof.

28. The suppressor of cell death in TDP-43-overexpressing cells according to claim 26, wherein the cycloserine is D-cycloserine.

29. The suppressor of cell death in TDP-43-overexpressing cells according to claim 26, wherein the cycloserine is L-cycloserine.

**30.** The suppressor of cell death in TDP-43-overexpressing cells according to claim 26, comprising 15 mg or more and less than 250 mg of at least one selected from the group consisting of cycloserine, terizidone, and salts thereof.

**31.** The suppressor of cell death in TDP-43-overexpressing cells according to claim 26, which is used in combination with 30 mg or more and 60 mg or less of the edaravone.

**32.** The suppressor of cell death in TDP-43-overexpressing cells according to claim 26, which does not comprise acamprosate.

**33.** A suppressor of cell death in TDP-43-overexpressing cells, the suppressor comprising edaravone used in combination with at least one selected from the group consisting of cycloserine, terizidone, and salts thereof.

**34.** The suppressor of cell death in TDP-43-overexpressing cells according to claim 33, comprising cycloserine or salt thereof.

**35.** The suppressor of cell death in TDP-43-overexpressing cells according to claim 33, wherein the cycloserine is D-cycloserine.

**36.** The suppressor of cell death in TDP-43-overexpressing cells according to claim 33, wherein the cycloserine is L-cycloserine.

**37.** The suppressor of cell death in TDP-43-overexpressing cells according to claim 33, which is used in combination with 15 mg or more and less than 250 mg of at least one selected from the group consisting of cycloserine, terizidone, and salts thereof.

**38.** The suppressor of cell death in TDP-43-overexpressing cells according to claim 33, wherein a content of the edaravone is 30 mg or more and 60 mg or less.

**39.** The suppressor of cell death in TDP-43-overexpressing cells according to claim 33, which does not comprise acamprosate.

**40.** An agent for treating or preventing diseases associated with TDP-43 aggregation, the agent comprising a combination of at least one selected from the group consisting of cycloserine, terizidone, and salts thereof, and edaravone.

**41.** The agent for treating or preventing diseases associated with TDP-43 aggregation according to claim 40, comprising cycloserine or salt thereof.

**42.** The agent for treating or preventing diseases associated with TDP-43 aggregation according to claim 40, wherein the cycloserine is D-cycloserine.

**43.** The agent for treating or preventing diseases associated with TDP-43 aggregation according to claim 40, wherein the cycloserine is L-cycloserine.

**44.** The agent for treating or preventing diseases associated with TDP-43 aggregation according to claim 40, wherein the disease associated with TDP-43 aggregation is TDP-43 proteinopathy.

**45.** The agent for treating or preventing diseases associated with TDP-43 aggregation according to claim 44, wherein the TDP-43 proteinopathy is at least one selected from the group consisting of amyotrophic lateral sclerosis, frontotemporal lobar degeneration, primary lateral sclerosis, and progressive muscular atrophy.

**46.** The agent for treating or preventing diseases associated with TDP-43 aggregation according to claim 44, wherein the TDP-43 proteinopathy is amyotrophic lateral sclerosis.

**47.** The agent for treating or preventing diseases associated with TDP-43 aggregation according to claim 40, comprising 15 mg or more and less than 250 mg of at least one selected from the group consisting of cycloserine, terizidone, and salts thereof.

**48.** The agent for treating or preventing diseases associated with TDP-43 aggregation according to claim 40, wherein a content of the edaravone is 30 mg or more and 60 mg or less.

**49.** The agent for treating or preventing diseases associated with TDP-43 aggregation according to claim 40, which does not comprise acamprosate.

**50.** An agent for treating or preventing diseases associated with TDP-43 aggregation, the agent comprising at least one selected from the group consisting of cycloserine, terizidone, and salts thereof, used in combination with edaravone.

**51.** The agent for treating or preventing diseases associated with TDP-43 aggregation according to claim 50, comprising cycloserine or salt thereof.

**52.** The agent for treating or preventing diseases associated with TDP-43 aggregation according to claim 50, wherein the cycloserine is D-cycloserine.

**53.** The agent for treating or preventing diseases associated with TDP-43 aggregation according to claim 50, wherein the cycloserine is L-cycloserine.

**54.** The agent for treating or preventing diseases associated with TDP-43 aggregation according to claim 50, wherein the disease associated with TDP-43 aggregation is TDP-43 proteinopathy.

**55.** The agent for treating or preventing diseases associated with TDP-43 aggregation according to claim 54, wherein the TDP-43 proteinopathy is at least one selected from the group consisting of amyotrophic lateral sclerosis, frontotemporal lobar degeneration, primary lateral sclerosis, and progressive muscular atrophy.

**56.** The agent for treating or preventing diseases associated with TDP-43 aggregation according to claim 54, wherein the TDP-43 proteinopathy is amyotrophic lateral sclerosis.

**57.** The agent for treating or preventing diseases associated with TDP-43 aggregation according to claim 50, comprising 15 mg or more and less than 250 mg of at least one selected from the group consisting of cycloserine, terizidone, and salts thereof.

**58.** The agent for treating or preventing diseases associated with TDP-43 aggregation according to claim 50, which is used in combination with 30 mg or more and 60 mg or less of the edaravone.

**59.** The agent for treating or preventing diseases associated with TDP-43 aggregation according to claim 50, which does not comprise acamprosate.

**60.** An agent for treating or preventing diseases associated with TDP-43 aggregation, the agent comprising edaravone used in combination with at least one selected from the group consisting of cycloserine, terizidone, and salts thereof.

**61.** The agent for treating or preventing diseases associated with TDP-43 aggregation according to claim 60, comprising cycloserine or salt thereof.

**62.** The agent for treating or preventing diseases associated with TDP-43 aggregation according to claim 60, wherein the cycloserine is D-cycloserine.

**63.** The agent for treating or preventing diseases associated with TDP-43 aggregation according to claim 60, wherein the cycloserine is L-cycloserine.

**64.** The agent for treating or preventing diseases associated with TDP-43 aggregation according to claim 60, wherein the disease associated with TDP-43 aggregation is TDP-43 proteinopathy.

**65.** The agent for treating or preventing diseases associated with TDP-43 aggregation according to claim 64, wherein the TDP-43 proteinopathy is at least one selected from the group consisting of amyotrophic lateral sclerosis, frontotemporal lobar degeneration, primary lateral sclerosis, and progressive muscular atrophy.

**66.** The agent for treating or preventing diseases associated with TDP-43 aggregation according to claim 64, wherein the TDP-43 proteinopathy is amyotrophic lateral sclerosis.

**67.** The agent for treating or preventing diseases associated with TDP-43 aggregation according to claim 60, which is used in combination with 15 mg or more and less than 250 mg of at least one selected from the group consisting of cycloserine, terizidone, and salts thereof.

**68.** The agent for treating or preventing diseases associated with TDP-43 aggregation according to claim 60, wherein a content of the edaravone is 30 mg or more and 60 mg or less.

**69.** The agent for treating or preventing diseases associated with TDP-43 aggregation according to claim 60, which does not comprise acamprosate.

Fig. 1

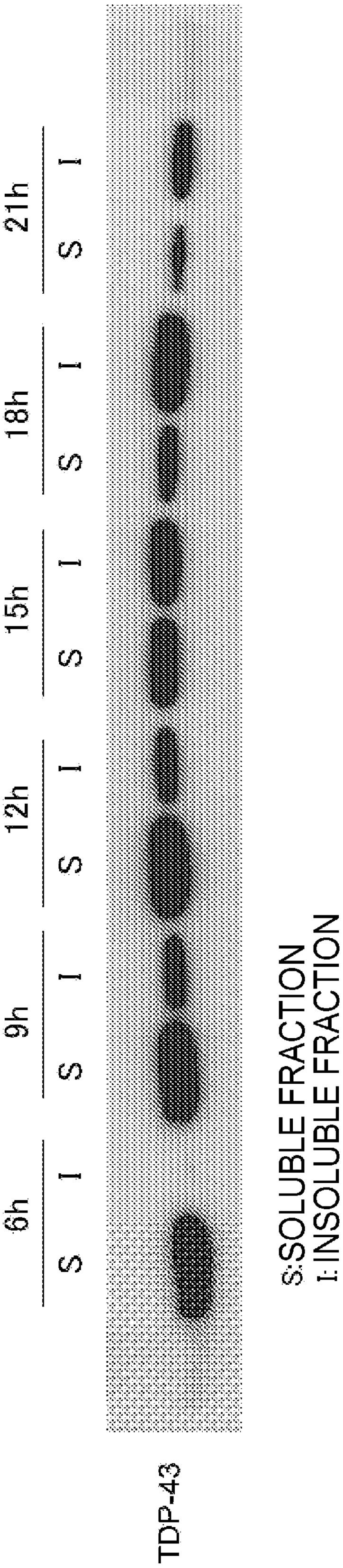
6h
9h
12h
15h
18h
21h
S I S I S I S I S I S I
TDP-43
S:SOLUBLE FRACTION
I: INSOLUBLE FRACTION

Fig. 2

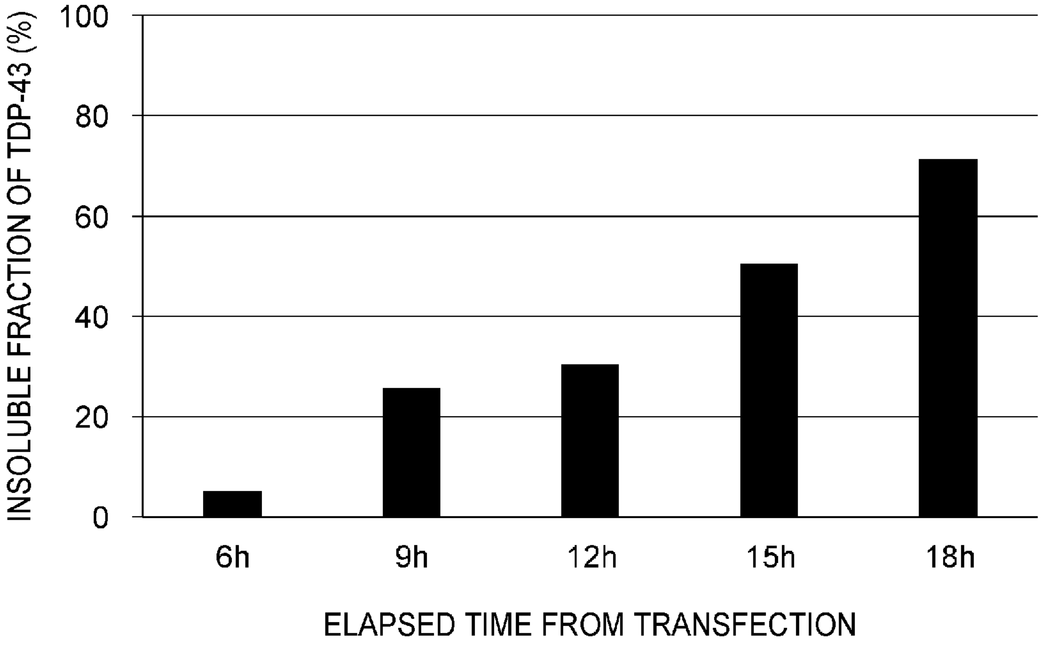
INSOLUBLE FRACTION OF TDP-43 (%)
100
80
60
40
20
0
6h
9h
12h
15h
18h
ELAPSED TIME FROM TRANSFECTION

# Fig. 3

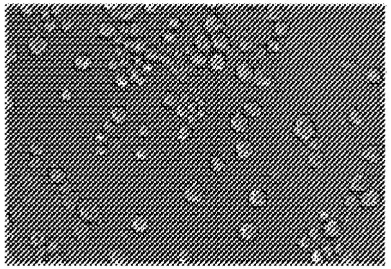

A WITHOUT TRANSFECTION

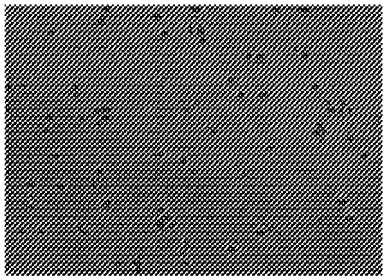

B WITH TRANSFECTION

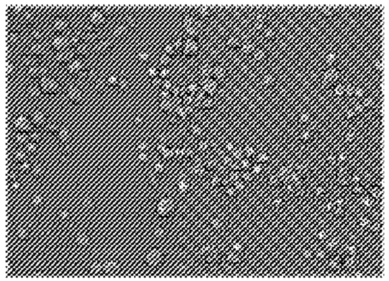

C WITH TRANSFECTION,
ADD 10 μmol/L OF D-cycloserine

Fig. 4

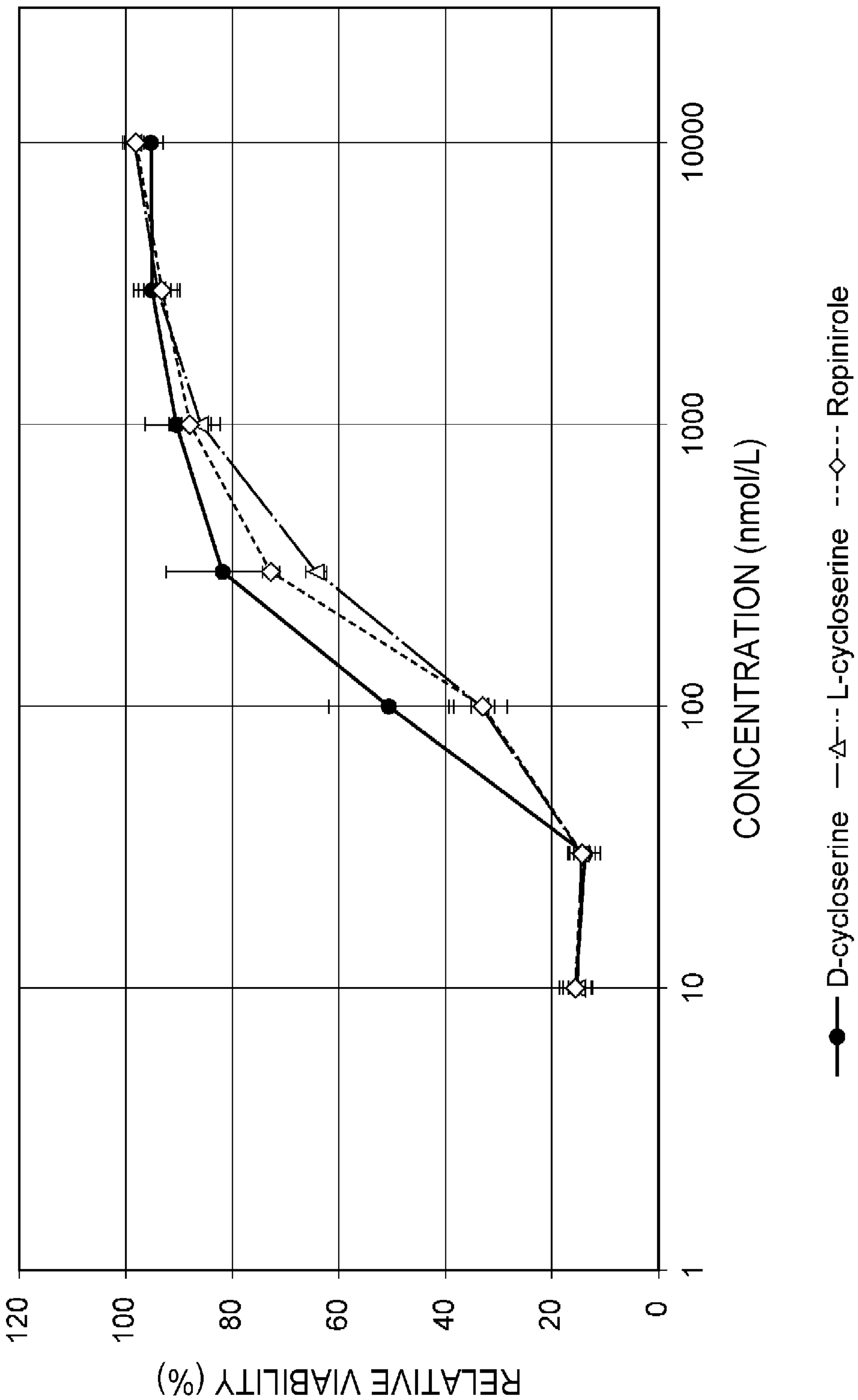

RELATIVE VIABILITY (%)
0
20
40
60
80
100
120
1
10
100
1000
10000
CONCENTRATION (nmol/L)
D-cycloserine
L-cycloserine
Ropinirole

# Fig. 5

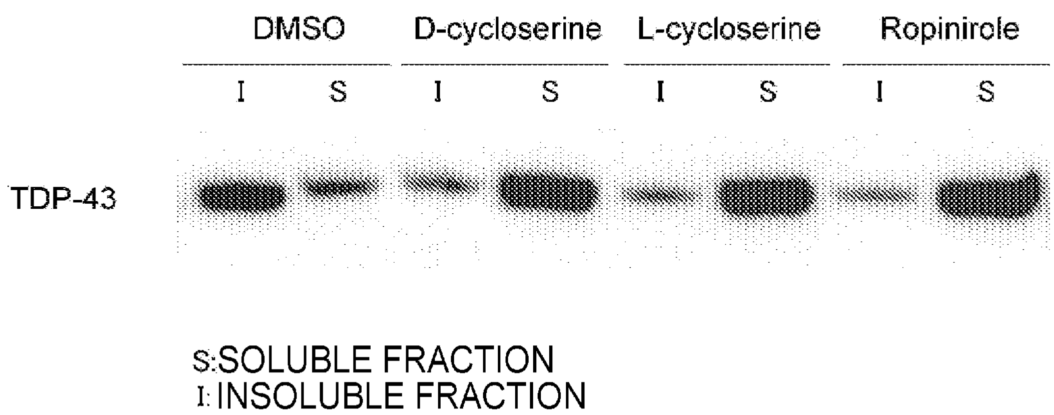
DMSO
D-cycloserine
L-cycloserine
Ropinirole
I
S
I
S
I
S
I
S
TDP-43
S:SOLUBLE FRACTION
I: INSOLUBLE FRACTION

# Fig. 6

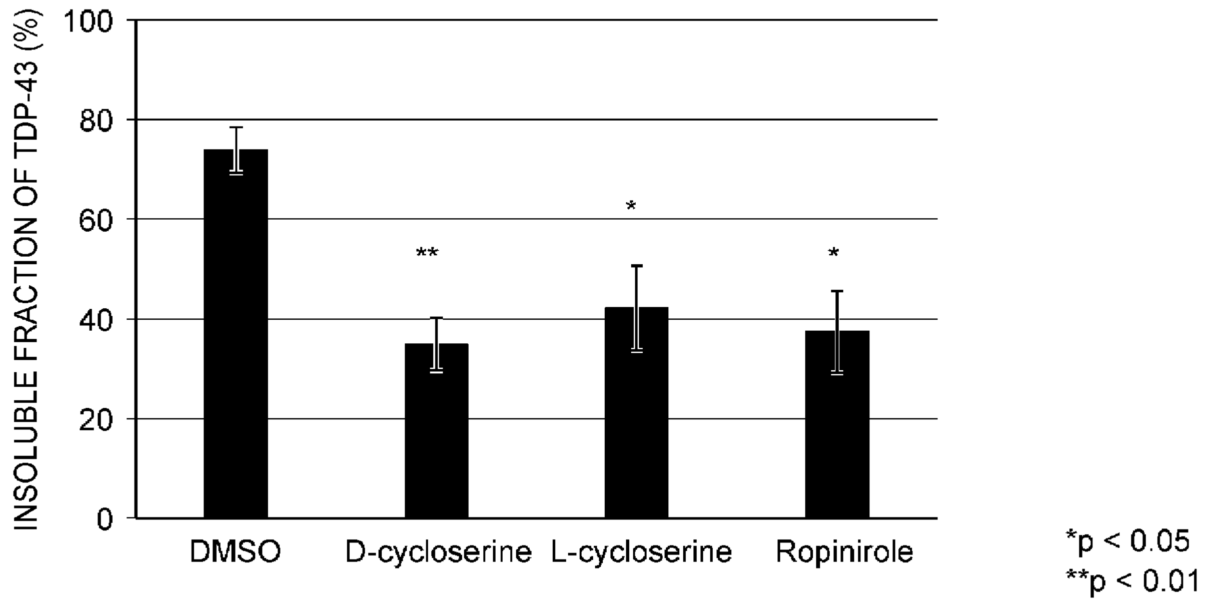


| | DMSO | D-cycloserine | L-cycloserine | Ropinirole |
|---|---|---|---|---|
| INSOLUBLE TDP-43 (%) | 73.87 | 34.93 | 42.19 | 37.42 |

Fig. 7

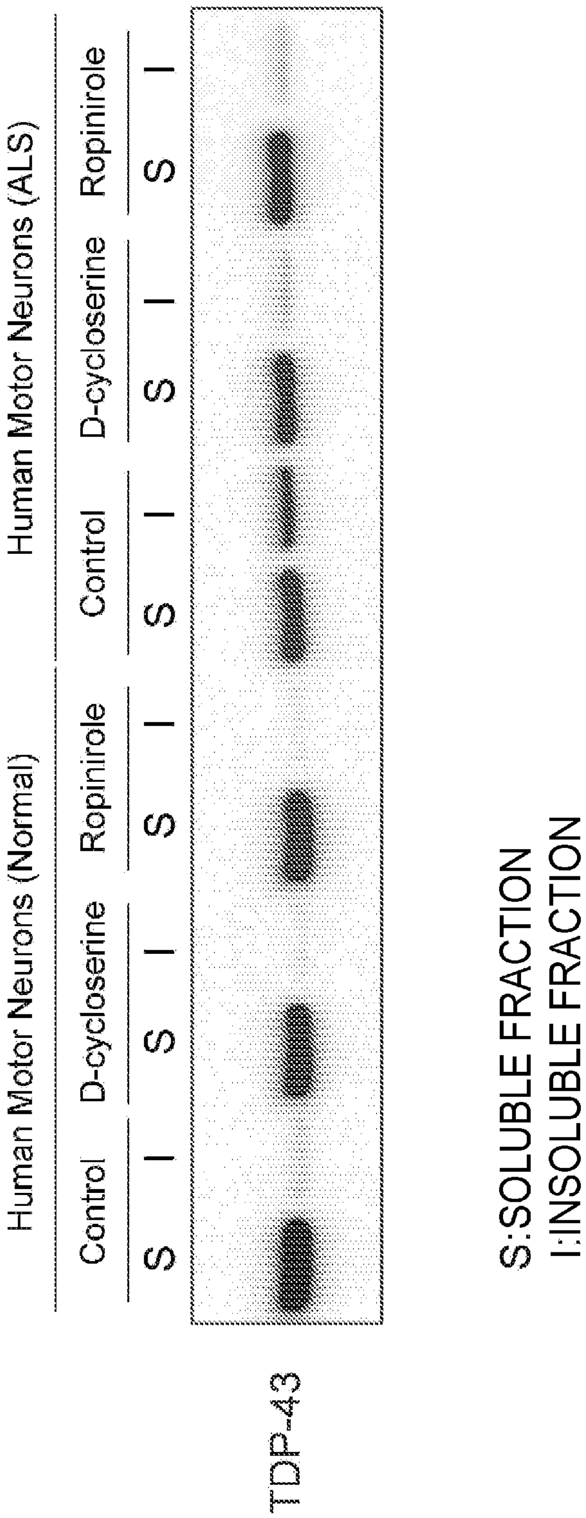

Human Motor Neurons (Normal)
Human Motor Neurons (ALS)
Control
D-cycloserine
Ropinirole
Control
D-cycloserine
Ropinirole
S I S I S I S I S I S I
TDP-43
S:SOLUBLE FRACTION
I:INSOLUBLE FRACTION

Fig. 8

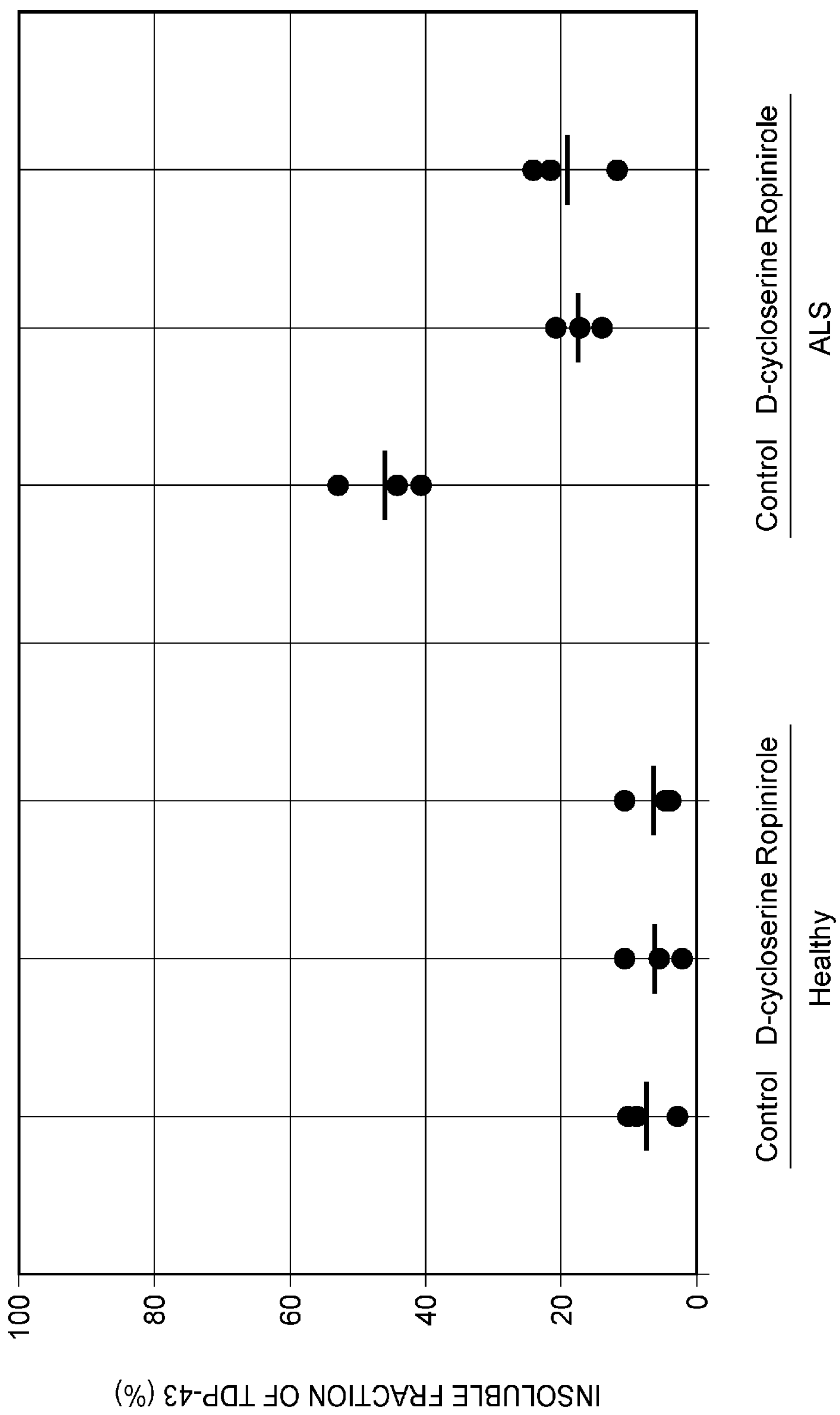
INSOLUBLE FRACTION OF TDP-43 (%)
100
80
60
40
20
0
Control
D-cycloserine
Ropinirole
Healthy
Control
D-cycloserine
Ropinirole
ALS

Fig. 9

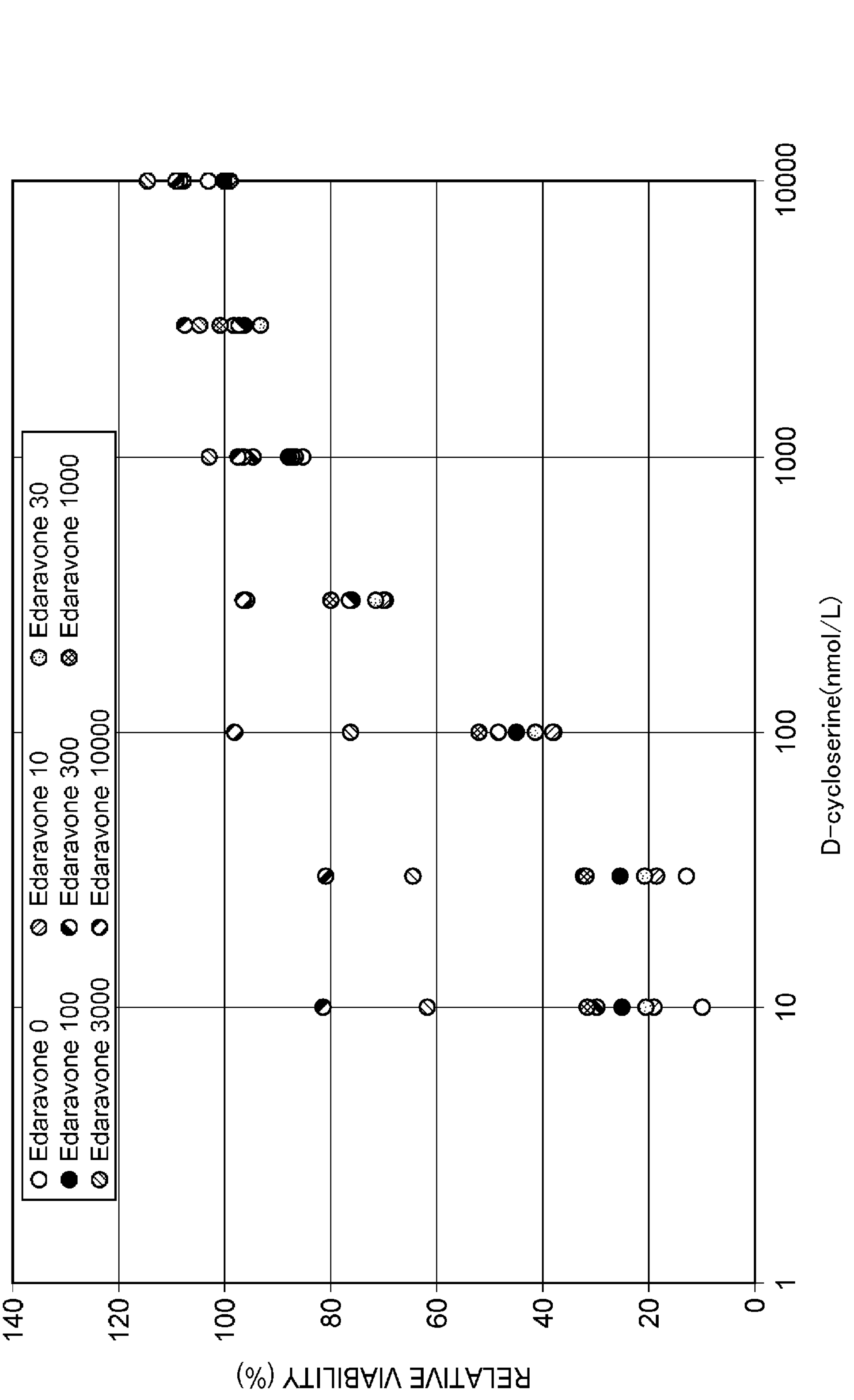


NUMERICAL VALUES FOLLOWING "Edaravone" IN GRAPH ALL (nmol/L)

Fig. 10

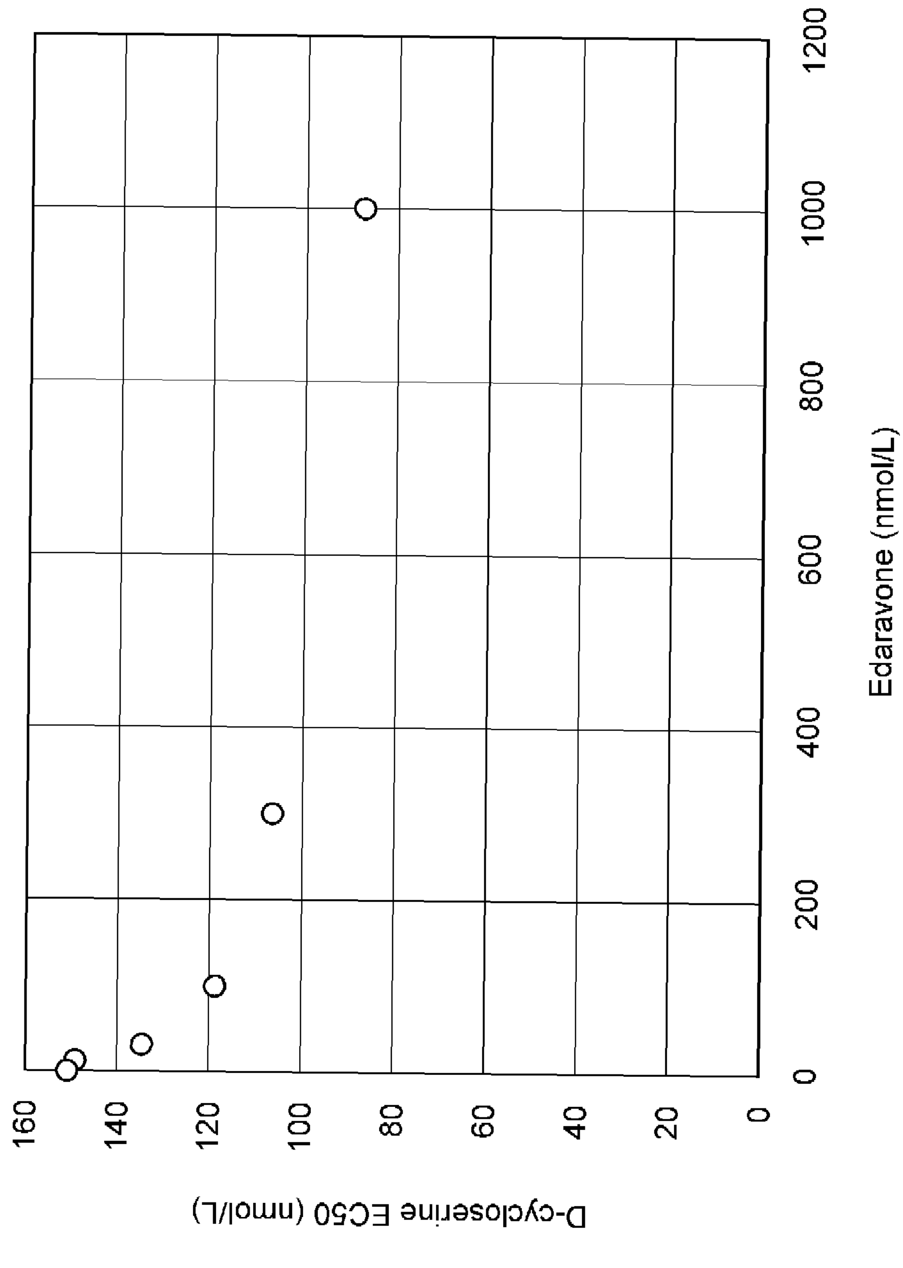

D-cycloserine EC50 (nmol/L)
160
140
120
100
80
60
40
20
0
200
400
600
800
1000
1200
Edaravone (nmol/L)

## Fig. 11

| Edaravone (nmol/L) | 0 | 10 | 30 | 100 | 300 | 1000 |
|---|---|---|---|---|---|---|
| D-cycloserine EC50 (nmol/L) | 151.36 | 149.44 | 135.18 | 118.85 | 106.76 | 87.62 |
| Combination Index | | 0.99 | 0.91 | 0.83 | 0.83 | 1.01 |

## INTERNATIONAL SEARCH REPORT

International application No.
**PCT/JP2024/027845**

### A. CLASSIFICATION OF SUBJECT MATTER

***A61K 31/42***(2006.01)i; ***A61K 31/422***(2006.01)i; ***A61K 31/4152***(2006.01)i; ***A61P 21/00***(2006.01)i; ***A61P 25/00***(2006.01)i; ***A61P 43/00***(2006.01)i
FI: A61K31/42; A61K31/4152; A61P43/00 111; A61P43/00 121; A61P21/00; A61P25/00; A61K31/422

According to International Patent Classification (IPC) or to both national classification and IPC

### B. FIELDS SEARCHED

Minimum documentation searched (classification system followed by classification symbols)

A61K31/42; A61K31/422; A61K31/4152; A61P21/00; A61P25/00; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | LAN, Weiss et al. Ceramide contributes to pathogenesis and may be targeted for therapy in VCP inclusion body myopathy. Hum. Mol. Genetics. 2020, vol. 29, no. 24, pp. 3945-3953<br>title, abstract, each drawing, each table, etc. | 1-69 |
| Y | SOEJIMA-KUSUNOKI, Aki et al. The Protective Effect of Edaravone on TDP-43 Plus Oxidative Stress-Induced Neurotoxicity in Neuronal Cells: Analysis of Its Neuroprotective Mechanisms Using RNA Sequencing. Pharmaceuticals. 2022, 15, 842., [online], [retrieved on 27 September 2024], URL [https://doi.org/10.3390/ph15070842]<br>title, abstract, fig. S3, etc., each drawing, each table, etc. | 1-69 |
| Y | COURT, R. et al. Steady state pharmacokinetics of cycloserine in patients on terizidone for multidrug-resistant tuberculosis. Int. J. Tuberc. Lung Dis. 2019, 22(1), [online], [retrieved on 27 September 2024], doi:10.5588/ijtld.17.0475.<br>p. 3, line 1 | 1-69 |

☐ Further documents are listed in the continuation of Box C. ☐ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 September 2024** | **08 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5898701 B

### Non-patent literature cited in the description


- *CHEMICAL ABSTRACTS*, 68-41-7
- *CHEMICAL ABSTRACTS*, 339-72-0
- *CHEMICAL ABSTRACTS*, 89-25-8
- *CHEMICAL ABSTRACTS*, 25683-71-0
- **VAN BERCKEL, B** ; **LIPSCH, C** ; **TIMP, S. et al.** Behavioral and Neuroendocrine Effects of the Partial NMDA Agonist D-cycloserine in Healthy Subjects.. *Neuropsychopharmacol*, 1997, vol. 16, 317-324, https://doi.org/10.1016/S0893-133X(96)00196-0
- **VAN BERCKEL** ; **B. ERRATUM**. Behavioral and Neuroendocrine Effects of the Partial NMDA Agonist D-cycloserine in Healthy Subjects.. *Neuropsychopharmacol*, 1997, vol. 17, 116, https://doi.org/10.1016/S0893-133X(97)00082-1
- **T C CHOU** ; **P TALALAY**. *Adv Enzyme Regul.*, 1984, vol. 22, 27-55